(19) Europäisches Patentamt
European Patent Office
Office européen des brevets

(11) EP 3 027 765 B1

(12) **EUROPEAN PATENT SPECIFICATION**

(45) Date of publication and mention
of the grant of the patent:
**10.07.2019 Bulletin 2019/28**

(51) Int Cl.:
**C12Q 1/18** (2006.01)     **G01N 33/02** (2006.01)

(21) Application number: **14761689.0**

(22) Date of filing: **30.07.2014**

(86) International application number:
**PCT/IB2014/001416**

(87) International publication number:
**WO 2015/015280 (05.02.2015 Gazette 2015/05)**

(54) **PRODUCTION OF NONTOXIC RAW MATERIALS AND FINISHED PRODUCTS TESTED BY MEANS OF AN INNOVATIVE PROBIOTIC BACTERIA BASED METHOD FOR DETERMINING TOXICITY TOWARDS PROBIOTIC BACTERIA**

HERSTELLUNG VON NICHTTOXISCHEN ROHSTOFFEN UND FERTIGPRODUKTEN SOWIE TESTS DAFÜR AUF DER BASIS EINES INNOVATIVEN PROBIOTISCHEN BAKTERIUMS ZUR BESTIMMUNG DER TOXIZITÄT GEGENÜBER PROBIOTISCHEN BAKTERIEN

PRODUCTION DE MATÉRIAUX BRUTS ET DE PRODUITS FINIS NON TOXIQUES SOUMIS À UN ESSAI AU MOYEN D'UN PROCÉDÉ INNOVANT À BASE DE BACTÉRIE PRO BIOTIQUE POUR DÉTERMINER LA TOXICITÉ ENVERS UNE BACTÉRIE PROBIOTIQUE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **30.07.2013 IT MI20131280**

(43) Date of publication of application:
**08.06.2016 Bulletin 2016/23**

(73) Proprietor: **Probiotical S.p.A.**
**28100 Novara (NO) (IT)**

(72) Inventor: **MOGNA, Giovanni**
**I-28100 Novara (IT)**

(74) Representative: **Hoffmann Eitle**
**Hoffmann Eitle S.R.L.**
**Piazza Sigmund Freud 1**
**20154 Milano (IT)**

(56) References cited:
**CN-A- 102 268 471     CN-A- 102 304 482**

• **DATABASE WPI Week 201259 Thomson Scientific, London, GB; AN 2012-K72233 XP002718118, & CN 102 552 525 A (UNIV HEBEI AGRIC) 11 July 2012 (2012-07-11)**

• **NARASIMHA RAO K ET AL: "INHIBITION OF THYMIDYLATE SYNTHASE AND CELL GROWTH BY THE PHENANTHROINDOLIZIDINE ALKALOIDS PERGULARININE AND TYLOPHORINIDINE", CHEMICO-BIOLOGICAL INTERACTIONS, vol. 106, no. 3, 24 October 1997 (1997-10-24), pages 201-212, XP008045242, ELSEVIER SCIENCE IRLAND, IR ISSN: 0009-2797, DOI: 10.1016/S0009-2797(97)00065-3**

• **LACKMAN-SMITH CAROL S ET AL: "Safety and anti-HIV assessments of natural vaginal cleansing products in an established topical microbicides in vitro testing algorithm", AIDS RESEARCH AND THERAPY, vol. 7, no. 1, 22, 9 July 2010 (2010-07-09), page 13PP, XP021079950, BIOMED CENTRAL, LONDON, GB ISSN: 1742-6405, DOI: 10.1186/1742-6405-7-22**

• **ANUKAM KINGSLEY C ET AL: "In vitro evaluation of the viability of vaginal cells (VK2/E6E7) and probiotic Lactobacillus species in lemon juice", SEXUAL HEALTH, COLLINGWOOD : CSIRO PUBL, vol. 6, no. 1, 1 March 2009 (2009-03-01), pages 67-74, XP009175157, ISSN: 1448-5028**

- ALI DEMIRCI ET AL: "Rapid screening of solvents and carrier compounds for lactic acid recovery by emulsion liquid extraction and toxicity on Lactobacillus casei (ATCC 11443)", BIOSEPARATION, vol. 7, no. 6, 1 November 1998 (1998-11-01), pages 297-308, XP019232354, KLUWER ACADEMIC PUBLISHERS, DO ISSN: 1573-8272
- FÉRIR GEOFFREY ET AL: "The lantibiotic peptide labyrinthopeptin A1 demonstrates broad anti-HIV and anti-HSV activity with potential for microbicidal applications.", PLOS ONE, vol. 8, no. 5, E64010, 28 May 2013 (2013-05-28), pages 1-16, XP002717956, ISSN: 1932-6203
- MONCLA B J ET AL: "Testing of viscous anti-HIV microbicides using Lactobacillus.", JOURNAL OF MICROBIOLOGICAL METHODS, vol. 88, no. 2, February 2012 (2012-02), pages 292-296, XP002717957, ISSN: 1872-8359
- MATSUMOTO M ET AL: "Extraction of organic acids using imidazolium-based ionic liquids and their toxicity to Lactobacillus rhamnosus", SEPARATION AND PURIFICATION TECHNOLOGY, vol. 40, no. 1, 15 November 2004 (2004-11-15), pages 97-101, XP004558244, ELSEVIER SCIENCE, AMSTERDAM, NL ISSN: 1383-5866, DOI: 10.1016/J.SEPPUR.2004.01.009
- C. LACKMAN-SMITH ET AL: "Development of a Comprehensive Human Immunodeficiency Virus Type 1 Screening Algorithm for Discovery and Preclinical Testing of Topical Microbicides", ANTIMICROBIAL AGENTS AND CHEMOTHERAPY, vol. 52, no. 5, 3 March 2008 (2008-03-03), pages 1768-1781, XP055093257, ISSN: 0066-4804, DOI: 10.1128/AAC.01328-07

**Description**

The present specification discloses a method for producing raw materials and of finished products intended for the food and pharmaceutical industries and devoid of toxicity, which is detectable by means of an innovative method - based on probiotic bacteria - for analyzing toxicity toward probiotic bacteria.

The documents CN 102304482A and CN 102268471A disclose a *Bifidobacterium longum* strain LJM002, that is ultra-sensitive to *acephate* and is a work strain for detecting the residue of this subatance in foods, and a microorganism method for screening the residue of *acephate* in foods.

All the materials or substances that are at the basis of the manufacture and production of other products through the use of appropriate processing and industrial processes that enable the desired final product to be obtained are considered raw materials.

The raw materials used in the production of food products or supplements or medical devices or pharmaceutical products include, by way of example, flavourings, extracts, co-formulants of organic and/or inorganic origin, technological additives, vitamins, proteins, amino acids, peptones, natural and/or synthetic polymers and still more.

By way of an illustrative and therefore non-exhaustive example, flavourings and/or extracts can be prepared from plants and/or their fruit.

It is well known that fruit-bearing plants and the fruit itself are today treated, for example, with chemical substances in order to protect them against the attacks of microorganisms or fungi or insects so as to enable the fruit to grow until reaching ripeness and then be harvested and consumed.

It is likewise well known that following said treatments, a part of the chemical substances used remains adsorbed on the exterior surface of the fruit, commonly called peel or rind. The chemical substances that are adsorbed also persist following successive washings of the fruit with water.

The peel (*exocarp*) is the protective outer layer of a fruit (epidermis, in turn made up of cuticle and sclerenchymatous tissue) or a vegetable which can be detached. The protective outer layer is also improperly called rind.

Furthermore, it cannot be ruled out that a part of the chemical substances adsorbed on the exterior surface of the fruit might penetrate into the fruit itself (into the flesh) as a result of absorption of said chemical substances from the outside toward the inside of the fruit.

Part of the fruit harvested is today used to produce natural vegetable extracts and/or flavourings (raw materials) that have application in the food, pharmaceutical and nutraceutical industries.

Usually, the natural vegetable extracts and/or flavourings are obtained by mechanical and/or chemical extraction from the whole fruit (peel and flesh) or from the peel and/or flesh treated separately, by means of the equipment and/or the techniques known to the person skilled in the art.

For example, in the case of flavourings and/or extracts and/or organic compounds obtained by extraction with solvents, it may occur that even by carrying out several washing steps with water and/or chemical solvents one does not succeed in completely eliminating all the solvent used; hence even a minimal presence can provoke toxicity to probiotic bacteria.

Therefore, it would be desirable to be able to have a method having high sensitivity and capable of identifying up until when there is a residual toxicity in a given substance or raw material in order to program the purification or washing or precipitation processes to be used to render the raw material free of toxicity to probiotic bacteria.

The same applies with reference to a protein extract or with reference to the preparation of amino acids. In this case as well, use is made of chemical reagents and/or solvents that can be left as residue and provoke toxicity to probiotic bacteria.

The same problem can also present itself with reference to inorganic raw materials, such as, for example silicon dioxide, widely used to formulate finished products.

With reference both to natural vegetable extracts and/or flavourings obtained by mechanical and/or chemical extraction and with reference to the other above-mentioned raw materials, prepared by means of processes of synthesis and/or extraction, one cannot disregard the fact that there may remain, within the raw material, minimal quantities of substances or compounds endowed with a "toxic" nature that impart a certain intrinsic toxicity to the raw material itself.

Therefore, with reference to natural vegetable extracts and/or with reference to the other above-mentioned raw materials, one cannot rule out the presence of toxic substances inside them in a variable quantity that can depend on the type of cultivation adopted for the fruit and/or the operating conditions adopted to carry out the mechanical and/or chemical extraction.

In any case, any toxic substances present in the natural vegetable extracts and/or flavourings and/or raw materials in general can give rise to two types of problems: an indirect one and a direct one.

The former, or indirect problem, is related to the influence that the intake of said toxic substances can have in altering the intestinal probiotic microflora and, therefore, also on the physiology of the digestive system, to such a point as to influence the absorption of vitamins, bioactive peptides and metabolites, and likewise permit the production of harmful biogenic amines, which are known to alter intestinal permeability and create a whole series of health problems, even arriving at the production of nitrosamine, well-known carcinogenic substances, said biogenic amines being produce by strains that have benefited from this alteration.

In this regard, it should be highlighted that, if the probiotic bacterial flora is altered as a result of an increase in the colonization of coliform pathogenic bacteria, such as, for example *E. coli,* endowed with decarboxylating properties capable of transforming an amino acid into an amine by eliminating the carboxyl group, abnormal quantities of harmful biogenic amines come to be produced.

Furthermore, an imbalance in intestinal microflora seems to be capable of contributing to the occurrence of various pathologies: diabetes and autoimmune diseases or, as has been hypothesized, to have a role in unbalanced local and systemic immune responses to certain food allergens.

The second, or direct problem, involves the effects that said toxic substances, already capable of killing the cells of probiotic bacteria, could have in individuals of paediatric age or in the development stage because of an immediate and/or accumulated toxic action, not only against bacterial cells but also against eukaryote cells, particularly sensitive in the differentiation and growth stage.

Thus there remains a need to be able to produce raw materials and finished products in an assuredly nontoxic manner and to have a method for determining the toxicity of an extract and/or a flavouring and/or a raw material in general that is sure, simple and practical to use, economical and repeatable.

In particular, there remains a need to have a method for determining the toxicity toward probiotic bacteria of the individual ingredients making up a finished product such as a food, a supplement, or a medical device or a drug.

The Applicant has found an innovative way of producing nontoxic raw materials and finished products, by subjecting the extracts and/or flavourings and/or raw materials in general to a new toxicity test in order to determine their toxicity toward probiotic bacteria.

The subject matter of the present invention is a method for producing nontoxic raw materials and finished products thanks to the possibility, provided by the Applicant, of being able to determine toxicity toward the probiotic bacteria present in said raw materials and finished products by means of an innovative method that likewise forms the subject matter of the present invention.

The Applicant has found that the toxicity determined with the method of the present invention depends not only on the raw material used per se, but also, and above all, on the type of mechanical and/or chemical extraction used to produce said raw material. In fact, the chemical extraction of a raw material can involve the use of chemical solvents, and some washing or precipitation steps that can leave residual toxicity in the raw material itself.

The method comprises a step in which the probiotic bacterial strain (toxicity marker) is placed in contact with a raw material to be tested using a quantity of raw material equal to that normally used in the formulation.

In practical terms, a first sample is prepared which comprises the probiotic bacterial strain (toxicity marker), the optimal culture substrate for said probiotic bacterial strain and the raw material to be tested. Then a second sample (internal reference) is prepared which comprises the same probiotic bacterial strain used in said first sample (toxicity marker) and only the optimal culture substrate (without the raw material to be tested).

The determination takes place by means of a bacterial plate count of said first sample and said second sample, as described below.

The ratio between the bacterial count (number of cells counted on the plate) of said first sample and the bacterial count (number of cells on the plate) of said second sample provides a number less than 1, which, if expressed as a percentage, provides a count of the bacteria which survived in contact with the raw material and, therefore, also expresses the % mortality induced by said raw material in the probiotic bacterial strains used as a marker.

A first embodiment relates to determining the toxicity of a raw material, such as, for example, a natural vegetable extract and/or flavouring and/or raw material in general.

In this case, the test of toxicity toward the probiotic bacteria entails setting up two tests in the laboratory, as described above.

In practical terms, a first sample is prepared which comprises the probiotic bacterial strain (toxicity marker), the optimal culture substrate for said probiotic bacterial strain and the raw material to be tested.

Then a second sample (internal reference) is prepared which comprises the same probiotic bacterial strain used in said first sample (toxicity marker) and only the optimal culture substrate (without the raw material to be tested).

The determination takes place by means of a bacterial plate count of said first sample and said second sample, as described below.

Preferably, said first and second test are performed in parallel under the same operating conditions.

The ratio between the bacterial count (number of cells counted on the plate) of said first sample and the bacterial count (number of cells on the plate) of said second sample provides a number less than 1, which, if expressed as a percentage, provides a count of the bacteria which survived in contact with the raw material and, therefore, also expresses the % mortality induced by said raw material in the probiotic bacterial strains used as a marker.

The difference between the bacterial count performed in said first test and the bacterial count performed in said second test provides a percentage of bacterial mortality which provides an indication of the toxicity toward probiotic bacteria associated with said raw material.

The Applicant, by way of non-exhaustive example, tested several flavourings and extracts present in the market, such as, for example, lemon and blueberry flavourings, which are also used in the preparation of finished products. The aim of these tests was to verify whether said foods or raw materials (lemon and blueberry flavourings) possessed an intrinsic toxicity toward probiotic bacteria.

For this reason, said raw materials (lemon and blueberry flavourings) were placed in contact with given strains of probiotic lactic bacteria or bifidobacteria under particular operating conditions.

The probiotic bacterial strains used as a toxicity marker in the method of the present invention belong to the species selected from the groups comprising lactobacilli and bifidobacteria. The present invention envisages the use of a bacterial strain among those listed in Table 1.

Table 1

| No. | Name | Commercial code | Depositary institution | Deposit number | Deposit date | Depositor |
|---|---|---|---|---|---|---|
| 1 | *Lactobacillus casei* | LF1i | CNCM I.P. | I-785 | 21.07.1988 | Anidral Srl |
| 2 | *Lactobacillus gasseri* | LF2i | CNCM I.P. | I-786 | 21.07.1988 | Anidral Srl |
| 3 | *Lactobacillus crispatus* | LF3i | CNCM IP. | I-787 | 21.07.1988 | Anidral Srl |
| 4 | *Lactobacillus fermentum* | LF4i | CNCM I.P. | I-788 | 21.07.1988 | Anidral Srl |
| 5 | *Lactobacillus fermentum* | LF5 | CNCM I.P. | I-789 | 21.07.1988 | Anidral Srl |
| 6 | *Lactobacillus casei* ssp. *pseudoplantarum* | LFH i | CNCM I.P. | I-790 | 21.07.1988 | Anidral Srl |
| 7 | *Streptococcus thermophilus* B39 | | BCCM LMG | LMG P-18383 | 5.05.1998 | Anidral Srl |
| 8 | *Streptococcus thermophilus* T003 | | BCCM LMG | LMG P-18384 | 5.05.1998 | Anidral Srl |
| 9 | *Lactobacillus pentosus* 9 /1 ei | | BCCM LMG | LMG P-21019 | 16.10.2001 | Mofin Srl |
| 10 | *Lactobacillus plantarum* 776 /1 bi | LP 02 | BCCM LMG | LMG P-21020 | 16.10.2001 | Mofin Srl |
| 11 | *Lactobacillus plantarum* 476LL 20 bi | LP 01 | BCCM LMG | LMG P-21021 | 16.10.2001 | Mofin Srl |
| 12 | *Lactobacillus plantarum* PR ci | | BCCM LMG | LMG P-21022 | 16.10.2001 | Mofin Srl |
| 13 | *Lactobacillus plantarum* 776 /2 hi | | BCCM LMG | LMG P-21023 | 16.10.2001 | Mofin Srl |
| 14 | *Lactobacillus casei* ssp. *paracasei* 181A/3 aiai | LPC00 | BCCM LMG | LMG P-21380 | 31.01.2002 | Anidral Srl |
| 15 | *Lactobacillus* belonging to the *acidophilus* group 192A/1 aiai | LA 02 | BCCM LMG | LMG P-21381 | 31.01.2002 | Anidral Srl |
| 16 | *Bifidobacterium longum* 175A/1 aiai | | BCCM LMG | LMG P-21382 | 31.01.2002 | Anidral Srl |
| 17 | *Bifidobacterium breve* 195A/1 aici | | BCCM LMG | LMG P-21383 | 31.01.2002 | Anidral Srl |
| 18 | *Bifidobacterium lactis* 32A/3 aiai | BS 01 | BCCM LMG | LMG P-21384 | 31.01.2002 | Anidral Srl |
| 19 | *Lactobacillus plantarum* 501/2 gi | COAKTIV | BCCM LMG | LMG P-21385 | 31.01.2002 | Mofin Srl |

(continued)

| No. | Name | Commercial code | Depositary institution | Deposit number | Deposit date | Depositor |
|---|---|---|---|---|---|---|
| 20 | *Lactococcus lactis* ssp. *lactis* 501/4 ci | | BCCM LMG | LMG P-21388 | 31.01.2002 | Mofin Srl |
| 21 | *Lactococcus lactis* ssp. *lactis* 501/4 hi | | BCCM LMG | LMG P-21387 | 15.03.2002 | Mofin Srl |
| 22 | *Lactococcus lactis* ssp. *lactis* 501/4 ci | | BCCM LMG | LMG P-21388 | 31.01.2002 | Mofin Srl |
| 23 | *Lactobacillus plantarum* 501/4 li | | BCCM LMG | LMG P-21389 | 15.03.2002 | Mofin Srl |
| 24 | *Lactobacillus acidophilus* | LA08 | BCCM LMG | LMG P-26144 | 03.11.2010 | Probiotical SpA |
| 25 | *Lactobacillus paracasei* ssp. *paracasei* | LPC10 | BCCM LMG | LMG P-26143 | 03.11.2010 | Probiotical SpA |
| 26 | *Streptococcus thermophilus* | GB1 | DSMZ | DSM 16506 | 18.06.2004 | Anidral Srl |
| 27 | *Streptococcus thermophilus* | GB5 | DSMZ | DSM 16507 | 18.06.2004 | Anidral Srl |
| 28 | *Streptococcus thermophilus* | Y02 | DSMZ | DSM 16590 | 20.07.2004 | Anidral Srl |
| 29 | *Streptococcus thermophilus* | Y03 | DSMZ | DSM 16591 | 20.07.2004 | Anidral Srl |
| 30 | *Streptococcus thermophilus* | Y04 | DSMZ | DSM 16592 | 20.07.2004 | Anidral Srl |
| 31 | *Streptococcus thermophilus* | YO5 | DSMZ | DSM 16593 | 20.07.2004 | Anidral Srl |
| 32 56 | *Bifidobacterium adolescentis* | BA 03 | DSMZ | DSM 16594 | 21.07.2004 | Anidral Srl |
| 33 | *Bifidobacterium adolescentis* | BA 04 | DSMZ | DSM 16595 | 21.07.2004 | Anidral Srl |
| 34 | *Bifidobacterium breve* | BR 04 | DSMZ | DSM 16596 | 21.07.2004 | Anidral Srl |
| 35 | *Bifidobacterium pseudocatenulatum* | BP 01 | DSMZ | DSM 16597 | 21.07.2004 | Anidral Srl |
| 36 | *Bifidobacterium pseudocatenulatum* | BP 02 | DSMZ | DSM 16598 | 21.07.2004 | Anidral Srl |
| 37 | *Bifidobacterium longum* | BL 03 | DSMZ | DSM 16603 | 20.07.2004 | Anidral Srl |
| 38 | *Bifidobacterium breve* | BR 03 | DSMZ | DSM 16604 | 20.07.2004 | Anidral Srl |
| 39 | *Lactobacillus casei* ssp. *rhamnosus* | LR 04 | DSMZ | DSM 16605 | 20.07.2004 | Anidral Srl |
| 40 | *Lactobacillus delbrueckii* ssp. *bulgaricus* | LDB 01 | DSMZ | DSM 16606 | 20.07.2004 | Anidral Srl |
| 41 | *Lactobacillus delbrueckii* ssp. *bulgaricus* | LDB 02 | DSMZ | DSM 16607 | 20.07.2004 | Anidral Srl |

(continued)

| No. | Name | Commercial code | Depositary institution | Deposit number | Deposit date | Depositor |
|---|---|---|---|---|---|---|
| 42 | *Staphylococcus xylosus* | SX 01 | DSMZ | DSM 17102 | 01.02.2005 | Anidral Srl |
| 43 = 57 | *Bifidobacterium adolescentis* | BA 02 | DSMZ | DSM 17103 | 01.02.2005 | Anidral Srl |
| 44 | *Lactobacillus plantarum* | LP 07 | DSMZ | DSM 17104 | 01.02.2005 | Anidral Srl |
| 45 | *Streptococcus thermophilus* | YO8 | DSMZ | DSM 17843 | 21.12.2005 | Anidral Srl |
| 46 | *Streptococcus thermophilus* | YO9 | DSMZ | DSM 17844 | 21.12.2005 | Anidral Srl |
| 47 | *Streptococcus thermophilus* | YO100 | DSMZ | DSM 17845 | 21.12.2005 | Anidral Srl |
| 48 | *Lactobacillus fermentum* | LF06 | DSMZ | DSM 18295 | 24.05.2006 | Anidral Srl |
| 49 | *Lactobacillus fermentum* | LF07 | DSMZ | DSM 18296 | 24.05.2006 | Anidral Srl |
| 50 | *Lactobacillus fermentum* | LF08 | DSMZ | DSM 18297 | 24.05.2006 | Anidral Srl |
| 51 | *Lactobacillus fermentum* | LF09 | DSMZ | DSM 18298 | 24.05.2006 | Anidral Srl |
| 52 | *Lactobacillus gasseri* | LGS01 | DSMZ | DSM 18299 | 24.05.2006 | Anidral Srl |
| 53 | *Lactobacillus gasseri* | LGS02 | DSMZ | DSM 18300 | 24.05.2006 | Anidral Srl |
| 54 | *Lactobacillus gasseri* | LGS03 | DSMZ | DSM 18301 | 24.05.2006 | Anidral Srl |
| 55 | *Lactobacillus gasseri* | LGS04 | DSMZ | DSM 18302 | 24.05.2006 | Anidral Srl |
| 56 = 32 | *Bifidobacterium adolescentis* EI-3 ***Bifidobacterium catenulatum sp./pseudocatenulatum* EI-3I, ID 09-255** | BA 03 | DSMZ | DSM 18350 | 15.06.2006 | Anidral Srl |
| 57 = 43 | *Bifidobacterium adolescentis* EI-15 | BA 02 | DSMZ | DSM 18351 | 15.06.2006 | Anidral Srl |
| 58 | *Bifidobacterium adolescentis* EI-18 ***Bifidobacterium animalis subsp. lactis* EI-18, ID 09-256** | BA 05 | DSMZ | DSM 18352 | 15.06.2006 | Anidral Srl |
| 59 | *Bifidobacterium catenulatum* EI-20 | BC 01 | DSMZ | DSM 18353 | 15.06.2006 | Anidral Srl |
| 60 | *Streptococcus thermophilus* FRai | MO1 | DSMZ | DSM 18613 | 13.09.2006 | Mofin Srl |
| 61 | *Streptococcus thermophilus* LB2bi | MO2 | DSMZ | DSM 18614 | 13.09.2006 | Mofin Srl |

(continued)

| No. | Name | Commercial code | Depositary institution | Deposit number | Deposit date | Depositor |
|-----|------|-----------------|------------------------|----------------|--------------|-----------|
| 62 | *Streptococcus thermophilus* LRci | MO3 | DSMZ | DSM 18615 | 13.09.2006 | Mofin Srl |
| 63 | *Streptococcus thermophilus* FP4 | MO4 | DSMZ | DSM 18616 | 13.09.2006 | Mofin Srl |
| 64 | *Streptococcus thermophilus* ZZ5F8 | MO5 | DSMZ | DSM 18617 | 13.09.2006 | Mofin Srl |
| 65 | *Streptococcus thermophilus* TEO4 | MO6 | DSMZ | DSM 18618 | 13.09.2006 | Mofin Srl |
| 66 | *Streptococcus thermophilus* S1ci | MO7 | DSMZ | DSM 18619 | 13.09.2006 | Mofin Srl |
| 67 | *Streptococcus thermophilus* 641bi | MO8 | DSMZ | DSM 18620 | 13.09.2006 | Mofin Srl |
| 68 | *Streptococcus thermophilus* 277A/1ai | MO9 | DSMZ | DSM 18621 | 13.09.2006 | Mofin Srl |
| 69 | *Streptococcus thermophilus* 277A/2ai | MO10 | DSMZ | DSM 18622 | 13.09.2006 | Mofin Srl |
| 70 | *Streptococcus thermophilus* IDC11 | MO11 | DSMZ | DSM 18623 | 13.09.2006 | Mofin Srl |
| 71 | *Streptococcus thermophilus* ML3di | MO14 | DSMZ | DSM 18624 | 13.09.2006 | Mofin Srl |
| 72 | *Streptococcus thermophilus* TEO3 | MO15 | DSMZ | DSM 18625 | 13.09.2006 | Mofin Srl |
| 73 | *Streptococcus thermophilus* G62 | GG1 | DSMZ | DSM 19057 | 21.02.2007 | Mofin Srl |
| 74 | *Streptococcus thermophilus* G1192 | GG2 | DSMZ | DSM 19058 | 21.02.2007 | Mofin Srl |
| 75 | *Streptococcus thermophilus* GB18 | GG3 MO2 | DSMZ | DSM 19059 | 21.02.2007 | Mofin Srl |
| 76 | *Streptococcus thermophilus* CCR21 | GG4 | DSMZ | DSM 19060 | 21.02.2007 | Mofin Srl |
| 77 | *Streptococcus thermophilus* G92 | GG5 | DSMZ | DSM 19061 | 21.02.2007 | Mofin Srl |
| 78 | *Streptococcus thermophilus* G69 | GG6 | DSMZ | DSM 19062 | 21.02.2007 | Mofin Srl |
| 79 | *Streptococcus thermophilus* | YO 10 | DSMZ | DSM 19063 | 21.02.2007 | Anidral Srl |
| 80 | *Streptococcus thermophilus* | YO 11 | DSMZ | DSM 19064 | 21.02.2007 | Anidral Srl |
| 81 | *Streptococcus thermophilus* | YO 12 | DSMZ | DSM 19065 | 21.02.2007 | Anidral Srl |
| 82 | *Streptococcus thermophilus* | YO 13 | DSMZ | DSM 19066 | 21.02.2007 | Anidral Srl |
| 83 | *Weissella* ssp. WSP 01 | EX | DSMZ | DSM 19067 | 21.02.2007 | Anidral Srl |

(continued)

| No. | Name | Commercial code | Depositary institution | Deposit number | Deposit date | Depositor |
|---|---|---|---|---|---|---|
| 84 | *Weissella* ssp. WSP 02 | EX | DSMZ | DSM 19068 | 21.02.2007 | Anidral Srl |
| 85 | *Lactobacillus* ssp. WSP 03 | EX | DSMZ | DSM 19069 | 21.02.2007 | Anidral Srl |
| 86 | *Lactobacillus plantarum* LP 09 | OY | DSMZ | DSM 19070 | 21.02.2007 | Anidral Srl |
| 87 | *Lactobacillus plantarum* LP 10 | OY | DSMZ | DSM 19071 | 21.02.2007 | Anidral Srl |
| 88 | *Lactococcus lactis* | NS 01 | DSMZ | DSM 19072 | 21.02.2007 | Anidral Srl |
| 89 | *Lactobacillus fermentum* | LF 10 | DSMZ | DSM 19187 | 20.03.2007 | Anidral Srl |
| 90 | *Lactobacillus fermentum* | LF 11 | DSMZ | DSM 19188 | 20.03.2007 | Anidral Srl |
| 91 | *Lactobacillus casei* ssp. *rhamnosus* | LR05 | DSMZ | DSM 19739 | 27.09.2007 | Anidral Srl |
| 92 | *Bifidobacterium bifidum* | BB01 | DSMZ | DSM 19818 | 30.10.2007 | Anidral Srl |
| 93 | *Lactobacillus delbrueckii* subsp. *bulgaricus* LD 01 | Lb | DSMZ | DSM 19948 | 28.11.2007 | Anidral Srl |
| 94 | *Lactobacillus delbrueckii* subsp. *bulgaricus* LD 02 | Lb | DSMZ | DSM 19949 | 28.11.2007 | Anidral Srl |
| 95 | *Lactobacillus delbrueckii* subsp. *bulgaricus* LD 03 | Lb | DSMZ | DSM 19950 | 28.11.2007 | Anidral Srl |
| 96 | *Lactobacillus delbrueckii* subsp. *bulgaricus* LD 04 | Lb | DSMZ | DSM 19951 | 28.11.2007 | Anidral Srl |
| 97 | *Lactobacillus delbrueckii* subsp. *bulgaricus* LD 05 | Lb | DSMZ | DSM 19952 | 28.11.2007 | Anidral Srl |
| 98 | *Bifidobacterium pseudocatenulatum* | B660 | DSMZ | DSM 21444 | 13.05.2008 | Probiotical SpA |
| 99 | *Lactobacillus acidophilus* | LA02 | DSMZ | DSM 21717 | 06.08.2008 | Probiotical SpA |
| 100 | *Lactobacillus paracasei* | LPC 08 | DSMZ | DSM 21718 | 06.08.2008 | Probiotical SpA |
| 101 | *Lactobacillus pentosus* | LPS 01 | DSMZ | DSM 21980 | 14.11.2008 | Probiotical SpA |
| 102 | *Lactobacillus rahmnosus* | LR 06 | DSMZ | DSM 21981 | 14.11.2008 | Probiotical SpA |
| 103 | *Lactobacillus delbrueckii* ssp. *delbrueckii* | DSMZ 20074 | DSMZ | DSM 22106 | 10.12.2008 | Probiotical SpA |
| 104 | *Lactobacillus plantarum* | LP1 | DSMZ | DSM 22107 | 10.12.2008 | Probiotical SpA |
| 105 | *Lactobacillus salivarius* | LS01 | DSMZ | DSM 22775 | 23.07.2009 | Probiotical SpA |

(continued)

| No. | Name | Commercial code | Depositary institution | Deposit number | Deposit date | Depositor |
|---|---|---|---|---|---|---|
| 106 | *Lactobacillus salivarius* | LS03 | DSMZ | DSM 22776 | 23.07.2009 | Probiotical SpA |
| 107 | *Bifidobacterium bifidum* | BB01 | DSMZ | DSM 22892 | 28.08.2009 | Probiotical SpA |
| 108 | *Bifidobacterium bifidum* | | DSMZ | DSM 22893 | 28.08.2009 | Probiotical SpA |
| 109 | *Bifidobacterium bifidum* | BB03 | DSMZ | DSM 22894 | 28.08.2009 | Probiotical SpA |
| 110 | *Bifidobacterium lactis* | BS05 | DSMZ | DSM 23032 | 13.10.2009 | Probiotical SpA |
| 111 | *Lactobacillus acidophilus* | LA 06 | DSMZ | DSM 23033 | 13.10.2009 | Probiotical SpA |
| 112 | *Lactobacillus brevis* | LBR01 | DSMZ | DSM 23034 | 13.10.2009 | Probiotical SpA |
| 113 | *Bifidobacterium animalis* ssp. *lactis* | BS06 | DSMZ | DSM 23224 | 12.01.2010 | Probiotical SpA |
| 114 | *Bifidobacterium longum* | BL04 | DSMZ | DSM 23233 | 12.01.2010 | Probiotical SpA |
| 115 | *Bifidobacterium longum* | BL05 | DSMZ | DSM 23234 | 12.01.2010 | Probiotical SpA |
| 116 | *Bifidobacterium bifidum* | MB 109 | DSMZ | DSM 23731 | 29.06.2010 | Probiotical SpA |
| 117 | *Bifidobacterium breve* | MB 113 | DSMZ | DSM 23732 | 29.06.2010 | Probiotical SpA |
| 118 | *Bifidobacterium lactis* | MB 2409 | DSMZ | DSM 23733 | 29.06.2010 | Probiotical SpA |
| 119 | *Lactobacillus reuteri* | LRE01 | DSMZ | DSM 23877 | 05.08.2010 | Probiotical SpA |
| 120 | *Lactobacillus reuteri* | LRE02 | DSMZ | DSM 23878 | 05.08.2010 | Probiotical SpA |
| 121 | *Lactobacillus reuteri* | LRE03 | DSMZ | DSM 23879 | 05.08.2010 | Probiotical SpA |
| 122 | *Lactobacillus reuteri* | LRE04 | DSMZ | DSM 23880 | 05.08.2010 | Probiotical SpA |
| 123 | *Lactobacillus paracasei* ssp. *paracasei* | LPC09 | DSMZ | DSM 24243 | 23.11.2010 | Probiotical SpA |
| 124 | *Lactobacillus acidophilus* | LA 07 | DSMZ | DSM 24303 | 23.11.2010 | Probiotical SpA |
| 125 | *Bifidobacterium bifidum* | BB04 | DSMZ | DSM 24437 | 04.01.2011 | Probiotical SpA |
| 126 | *Lactobacillus crispatus* | CRL 1251 | DSMZ | DSM 24438 | 04.01.2011 | Probiotical SpA |
| 127 | *Lactobacillus crispatus* | CRL 1266 | DSMZ | DSM 24439 | 04.01.2011 | Probiotical SpA |

(continued)

| No. | Name | Commercial code | Depositary institution | Deposit number | Deposit date | Depositor |
|---|---|---|---|---|---|---|
| 128 | *Lactobacillus paracasei* | CRL 1289 | DSMZ | DSM 24440 | 04.01.2011 | Probiotical SpA |
| 129 | *Lactobacillus salivarius* | CRL 1328 | DSMZ | DSM 24441 | 04.01.2011 | Probiotical SpA |
| 130 | *Lactobacillus gasseri* | CRL 1259 | DSMZ | DSM 24512 | 25.01.2011 | Probiotical SpA |
| 131 | *Lactobacillus acidophilus* | CRL 1294 | DSMZ | DSM 24513 | 25.01.2011 | Probiotical SpA |
| 132 | *Lactobacillus salivarius* | LS04 | DSMZ | DSM 24618 | 02.03.2011 | Probiotical SpA |
| 133 | *Lactobacillus crispatus* | LCR01 | DSMZ | DSM 24619 | 02.03.2011 | Probiotical SpA |
| 134 | *Lactobacillus crispatus* | LCR02 | DSMZ | DSM 24620 | 02.03.2011 | Probiotical SpA |
| 135 | *Lacotbacillus acidophilus* | LA09 | DSMZ | DSM 24621 | 02.03.2011 | Probiotical SpA |
| 136 | *Lactobacillus gasseri* | LGS05 | DSMZ | DSM 24622 | 02.03.2011 | Probiotical SpA |
| 137 | *Lactobacillus paracasei* | LPC11 | DSMZ | DSM 24623 | 02.03.2011 | Probiotical SpA |
| 138 | *Bifidobacterium infantis* | BI 02 | DSMZ | DSM 24687 | 29.03.2011 | Probiotical SpA |
| 139 | *Bifidobacterium bifidum* | BB 06 | DSMZ | DSM 24688 | 29.03.2011 | Probiotical SpA |
| 140 | *Bifidobacterium longum* | BL 06 | DSMZ | DSM 24689 | 29.03.2011 | Probiotical SpA |
| 141 | *Bifidobacterium lactis* | BS 07 | DSMZ | DSM 24690 | 29.03.2011 | Probiotical SpA |
| 142 | *Bifidobacterium longum* | PCB133 | DSMZ | DSM 24691 | 29.03.2011 | Probiotical SpA |
| 143 | *Bifidobacterium breve* | B632 | DSMZ | DSM 24706 | 07.04.2011 | Probiotical SpA |
| 144 | *Bifidobacterium breve* | B2274 | DSMZ | DSM 24707 | 07.04.2011 | Probiotical SpA |
| 145 | *Bifidobacterium breve* | B7840 | DSMZ | DSM 24708 | 07.04.2011 | Probiotical SpA |
| 146 | *Bifidobacterium longum* | B1975 | DSMZ | DSM 24709 | 07.04.2011 | Probiotical SpA |
| 147 | *Lactobacillus salivarius* | DLV1 | DSMZ | DSM 25138 | 02.09.2011 | Probiotical SpA |
| 148 | *Lactobacillus reuteri* | LRE05 | DSMZ | DSM 25139 | 02.09.2011 | Probiotical SpA |

(continued)

| No. | Name | Commercial code | Depositary institution | Deposit number | Deposit date | Depositor |
|---|---|---|---|---|---|---|
| 149 | *Lactobacillus reuteri* | LRE06 | DSMZ | DSM 25140 | 02.09.2011 | Probiotical SpA |
| 150 | *Lactobacillus reuteri* | RC 14 | DSMZ | DSM 25141 | 02.09.2011 | Probiotical SpA |
| 151 | *Streptococcus thermophilus* | ST 10 | DSMZ | DSM 25246 | 19.09.2011 | Probiotical SpA |
| 152 | *Streptococcus thermophilus* | ST 11 | DSMZ | DSM 25247 | 19.09.2011 | Probiotical SpA |
| 153 | *Streptococcus thermophilus* | ST 12 | DSMZ | DSM 25282 | 20.10.2011 | Probiotical SpA |
| 154 | *Lactobacillus salivarius* | DLV8 | DSMZ | DSM 25545 | 12.01.2012 | Probiotical SpA |
| 155 | *Bifidobacterium longum* | DLBL 07 | DSMZ | DSM 25669 | 16.02.2012 | Probiotical SpA |
| 156 | *Bifidobacterium longum* | DLBL 08 | DSMZ | DSM 25670 | 16.02.2012 | Probiotical SpA |
| 157 | *Bifidobacterium longum* | DLBL 09 | DSMZ | DSM 25671 | 16.02.2012 | Probiotical SpA |
| 158 | *Bifidobacterium longum* | DLBL 10 | DSMZ | DSM 25672 | 16.02.2012 | Probiotical SpA |
| 159 | *Bifidobacterium longum* | DLBL 11 | DSMZ | DSM 25673 | 16.02.2012 | Probiotical SpA |
| 160 | *Bifidobacterium longum* | DLBL 12 | DSMZ | DSM 25674 | 16.02.2012 | Probiotical SpA |
| 161 | *Bifidobacterium longum* | DLBL13 | DSMZ | DSM 25675 | 16.02.2012 | Probiotical SpA |
| 162 | *Bifidobacterium longum* | DLBL 14 | DSMZ | DSM 25676 | 16.02.2012 | Probiotical SpA |
| 163 | *Bifidobacterium longum* | DLBL 15 | DSMZ | DSM 25677 | 16.02.2012 | Probiotical SpA |
| 164 | *Bifidobacterium longum* | DLBL 16 | DSMZ | DSM 25678 | 16.02.2012 | Probiotical SpA |
| 165 | *Bifidobacterium longum* | DLBL 17 | DSMZ | DSM 25679 | 16.02.2012 | Probiotical SpA |
| 166 | *Lactobacillus johnsonii* | DLLJO 01 | DSMZ | DSM 25680 | 16.02.2012 | Probiotical SpA |
| 167 | *Lactobacillus rhamnosus* | DLLR 07 | DSMZ | DSM 25681 | 16.02.2012 | Probiotical SpA |
| 168 | *Lactobacillus rhamnosus* | DLLR 08 | DSMZ | DSM 25682 | 16.02.2012 | Probiotical SpA |
| 169 | *Lactobacillus reuteri* | DLLRE 07 | DSMZ | DSM 25683 | 16.02.2012 | Probiotical SpA |

(continued)

| No. | Name | Commercial code | Depositary institution | Deposit number | Deposit date | Depositor |
|---|---|---|---|---|---|---|
| 170 | *Lactobacillus reuteri* | DLLRE 08 | DSMZ | DSM 25684 | 16.02.2012 | Probiotical SpA |
| 171 | *Lactobacillus reuteri* | DLLRE 09 | DSMZ | DSM 25685 | 16.02.2012 | Probiotical SpA |
| 172 | *Bifidobacterium longum* | DLBL 18 | DSMZ | DSM 25708 | 24.02.2012 | Probiotical SpA |
| 173 | *Bifidobacterium infantis* | BI 03 | DSMZ | DSM 25709 | 24.02.2012 | Probiotical SpA |
| 174 | *Lactobacillus plantarum* | LP 09 | DSMZ | DSM 25710 | 24.02.2012 | Probiotical SpA |
| 175 | *Bifidobacterium longum* | DLBL 19 | DSMZ | DSM 25717 | 01.03.2012 | Probiotical SpA |
| 176 | *Bifidobacterium longum* | DLBL 20 | DSMZ | DSM 25718 | 01.03.2012 | Probiotical SpA |
| 177 | *Lactobacillus salivarius* | LS 05 | DSMZ | DSM 26036 | 06.06.2012 | Probiotical SpA |
| 178 | *Lactobacillus salivarius* | LS 06 | DSMZ | DSM 26037 | 06.06.2012 | Probiotical SpA |
| 179 | *Lactobacillus pentosus* | LPS 02 | DSMZ | DSM 26038 | 06.06.2012 | Probiotical SpA |
| 180 | *Bifidobacterium pseudolongum* ssp. *globosum* | BPS 01 | DSMZ | DSM 26456 | 02.10.2012 | Probiotical SpA |
| 181 | *Lactobacillus fermentum* | LF15 | DSMZ | DSM 26955 | 01.03.2013 | Probiotical SpA |
| 182 | *Lactobacillus fermentum* | LF16 | DSMZ | DSM 26956 | 01.03.2013 | Probiotical SpA |
| 183 | *Lactobacillus casei* | LC03 | DSMZ | DSM 27537 | 24.07.2013 | Probiotical SpA |
| 184 | *Lactobacillus crispatus* | LCR03 | DSMZ | DSM 27538 | 24.07.2013 | Probiotical SpA |
| 185 | *Lactobacillus jensenii* | LJE01 | DSMZ | DSM 27539 | 24.07.2013 | Probiotical SpA |

The tests performed on said foods or raw materials (lemon and blueberry flavourings) showed an acute toxicity toward the probiotic bacterial strains used as toxicity markers. In practical terms, two lemon extracts (raw material) and two blueberry extracts of (raw material) from different suppliers were tested. It was possible to verify that a first lemon extract and a second blueberry one provoked an acute toxicity, 57% and 58% mortality respectively, toward the probiotic bacterial strains used having an initial theoretical load of $6 \times 10^9$ CFU/g. The tests were performed using the probiotic bacterial strain *Lactobacillus acidophilus* LA02 LMG P-21381 deposited by the company Anidral Srl on 31.01.2002, and the probiotic bacterial strain *Bifidobacterium animalis subsp. Lactis* BS01 LMG P-21384 deposited by the company Anidral Srl on 31.01.2002.

At this point the Applicant replicated the aforesaid tests using, in place of the blueberry and lemon flavourings present in the market, flavourings obtained only from the flesh of the fruit (lemon and blueberry) by gentle squeezing. In practical terms, in the case of both lemon and blueberry, the peel was separated from the flesh beforehand and only the latter was subjected to mechanical extraction under gentle conditions, using equipment and methods known to the person skilled in the art. In this case, with the extracts/raw materials obtained from an extraction of the flesh under gentle conditions, toxicity toward the probiotic bacteria was found to be inexistent; in fact, using an initial load of the probiotic bacterial strain equal to $6.7 \times 10^9$ CFU/g, $6.5 \times 10^9$ CFU/g were obtained with blueberry juice and $6.4 \times 10^9$ CFU/g with lemon juice, hence much better values than in the previous case with very low toxicity and a mortality close to zero.

Other raw materials were tested in the same way as the lemon and blueberry extracts, as described below.

The Applicant tested several finished products present in the market with the aim of determining the degree of toxicity toward probiotic bacteria and identify, among the raw materials used in said finished products, which of the raw materials used contributed most to the toxicity.

In a preferred embodiment, the toxicity of a raw material toward probiotic bacteria is determined, the raw material being for example an extract and/or a flavouring that is within a formulation of a finished product having, for example, a total of 5 components.

In this case, the toxicity test involves setting up a number of tests in the laboratory equal to the number of components - in this exemplifying case there are 5 components, plus the analytical references.

In order to show the potentiality of the present invention, the Applicant tested a series of samples of cranberry extract to be used in the preparation of a finished product, for example P1.

The toxicity of the raw material, cranberry extract (lot L1, lot L2, from four different suppliers) which must be present together with other components/ingredients within a finished product (P1) was determined.

In this case, two tests were set up. In a first test, a mixture consisting of a probiotic bacterial strain, for example LS01 (used as the toxicity marker) was prepared. This first test represents the internal analytical reference -Ref. 1. The expected theoretical bacterial count was equal to 25 MLD/dose (internal reference).

In a second test, a mixture containing the probiotic bacterial strain LS01 (used as the toxicity marker) together with a first cranberry extract - L1 and L2, in said finished product, from different suppliers, was prepared.

Said first and second bacterial counts were performed in parallel under the same operating conditions and with the same amounts as those present in said finished product.

Subsequently, the real bacterial count of said first test -Ref. 1 and the bacterial count of said second test were determined. The bacterial count was carried out adopting the same operating conditions.

Supplier V: lot 1 (L1) and lot 2 (L2) -Var cranberry

Supplier V: lot 1 (L1) and lot 2 (L2) -Var cranberry

Supplier K: lot 1 (L1) and lot 2 (L2) -Kem cranberry

Supplier K: lot 1 (L1) and lot 2 (L2) -Kem cranberry

Supplier P: lot 1 (L1) and lot 2 (L2) -Pac cranberry

Supplier P: lot 1 (L1) and lot 2 (L2) -Pac cranberry

Supplier N: lot 1 (L1) and lot 2 (L2) -Nut cranberry

Supplier N: lot 1 (L1) and lot 2 (L2) -Nut cranberry

The various supplies (lots) of the raw material, cranberry, were introduced into the mixture for the toxicity test based on their proanthocyanidin content (at least 1.5% (HPLC)), so at to ensure the same concentration of that ingredient in the finished product P1.

Table 2

| Mixture to be tested | Lot | Load MLD/dose | % mortality vs Ref. 1 |
|---|---|---|---|
| Bacterial strain LS01 (real load -Ref. 1) | | 25 | |
| Mixture: bacterial strain LS01+ Var cranberry extract [Supplier V] | L1 | 1.2 | 95 |
| Mixture: bacterial strain LS01 + Var cranberry extract [Supplier V] | L2 | 0.9 | 96 |
| Mixture: bacterial strain LS01 + Kem cranberry extract [Supplier K] | L1 | 0.8 | 97 |
| Mixture: bacterial strain LS01 + Kem cranberry extract [Supplier K] | L2 | 2 | 91 |
| Bacterial strain LS01 (real load -Ref. 1) | | 22 | |

(continued)

| Mixture to be tested | Lot | Load MLD/dose | % mortality vs Ref. 1 |
|---|---|---|---|
| Mixture: bacterial strain LS01 + Pac cranberry extract [Supplier P] | L1 | 20 | 9 |
| Mixture: bacterial strain LS01 + Pac cranberry extract [Supplier P] | L2 | 20 | 9 |
| Bacterial strain LS01 (real load -Ref. 1) | | 20 | |
| Mixture: bacterial strain LS01 + Nut cranberry extract [Supplier N] | L1 | 14 | 30 |
| Mixture: bacterial strain LS01 + Nut cranberry extract [Supplier N] | L2 | 9 | 55 |

The tests of Table 2 were also repeated with the probiotic bacterial strains indicated in Table 1 with the numbers 9, 33, 39, 46, 54, 59, 73, 84, 95, 101, 116, 130, 138, 143, 169 and 185 and the results obtained were very similar to those shown in Table 2.

The difference between the bacterial count performed in said first test -Ref.1 and the bacterial count performed in said second test provides a percentage of mortality of the probiotic bacterial strain LS01, which provides an indication of the toxicity of said cranberry extract towards said strain.

The viable cell load obtained from the bacterial count, as determined in said first test and in said second test, makes it possible to establish whether the tested raw material exerts a toxic effect on the cells of the probiotic bacterial strain LS01 present in said finished product P1.

The percentage decrease in the bacterial count compared to the reference -Ref.1 is expressed as % mortality induced by the raw material subjected to the toxicity test of the present invention.

The Applicant further applied the above-described method to lemon and blueberry extracts present in a finished product, obtaining results comparable to the ones obtained above with cranberry.

Table 2 shows that there are extracts, for example cranberry extract, which is commonly used to formulate finished products - but the same consideration also applies with reference to other raw materials - which impart toxicity to finished products and, consequently, also to the body of individuals who use said finished products.

Therefore, with the present invention it is possible to develop formulations of finished products such as dietary supplements or medical devices or pharmaceutical products devoid of toxicity or with greatly reduced toxicity, since it is possible to identify whether the raw materials used impart toxicity toward probiotic bacteria.

In the context of the present invention a percentage decrease in the bacterial load compared to the internal reference [(Bacterial count of the test sample)/(Bacterial count of the internal reference)] = % mortality induced by the raw material comprised from 1 to 5% signifies no mortality; a value comprised from greater than 5 to 15% signifies a low mortality, still acceptable; a value comprised from greater than 15 to 25% signifies medium-high mortality, whereas beyond 25 % we are facing acute mortality.

The method of the present invention has valid application, for example, in testing for the presence of toxic excipients or raw materials used in the formulations of supplements and medical devices (finished products), such as, for example, orange flavouring, safflower, black carrot, blueberry extract etc.).

The method of the present invention is illustrated below by way of example and therefore does not limit of the scope of the present invention.

In a preferred embodiment, in the case of a finished product containing, for example, the following components A, B, C (complete formulation A+B+C), the method of the present invention can be used in order to determine whether the finished product as a whole (A+B+C) exerts toxicity towards the probiotic bacterial strains.

In this case, the test of the toxicity toward the probiotic bacteria involves setting up two tests in the laboratory.

In practical terms, a first sample is prepared which comprises the probiotic bacterial strain (toxicity marker), the optimal culture substrate for said probiotic bacterial strain and the raw material to be tested, in this case the formulation A+B+C.

Then a second sample (internal reference) is prepared which comprises the same probiotic bacterial strain used in said first sample (toxicity marker) and only the optimal culture substrate (without the raw material to be tested).

The determination takes place by means of a bacterial plate count of said first sample and said second sample.

Preferably, said first and second tests are performed in parallel under the same operating conditions.

If the ratio between the bacterial count (number of cells counted on the plate) of said first sample and the bacterial count (number of cells counted on the plate) of said second sample provides a number lower than 1, for example 0.55 (or 55%), it means that the count of the bacteria that have survived in contact with the raw material is 55% and, therefore, the % mortality induced by A+B+C in the probiotic bacterial strain used as the marker is 45%.

If, precisely, a toxicity toward the probiotic bacterial strain used as a marker emerges, the next step is to go and determine which of the components A, B and C making up the finished product (A+B+C), exerts the detected toxicity.

In this case, the method involves preparing three tests (test 1, test 2 and test 3) as specified below.

For example, test 1 is performed on raw material A and involves preparing the following samples:

(i) A first sample is prepared which comprises the probiotic bacterial strain (toxicity marker), the optimal culture substrate for said probiotic bacterial strain and the raw material to be tested, in this case A.

(ii) A second sample (internal reference) is prepared which comprises the same probiotic bacterial strain used in said first sample (toxicity marker) and only the optimal culture substrate (without the raw material to be tested).

(iii) A bacterial plate count is performed on said first sample and said second sample.

(iv) The percentage of mortality is determined.

Analogously, test 2 with raw material B and test 3 with raw material C are prepared with the same method. In this manner, it is possible to identify which, among the components A, B and C present in the formulation A+B+C, is the component that exerts toxicity towards probiotic bacterial strains. The toxicity tests are performed under the same operating conditions and at the concentrations indicated in the finished product-sequential approach.

For the plate count one follows the method, described below by way of non-limiting example of a test method, which comprises a traditional microbiological count with initial resuspension of the sample, serial dilutions in a suitable diluent, plating on an agarized medium and a colony count after incubation under optimal conditions. The excipients/raw materials that determine a plate mortality comparable with that of the reference sample are to be considered as conforming (reduced toxicity or no toxicity whatsoever).

As noted above, one performs a total, differential and/or selective (in an agarized medium) count of lactic bacteria and bifidobacteria for probiotic use, present alone or in admixture in the sample to be subjected to a determination of the concentration of live, viable cells.

The formulation of the culture medium is such as to ensure the growth of all the various species of probiotic bacteria belonging to the aforesaid microbial groups and if necessary to enable them to be discriminated by adding selective agents (generally antibiotics and/or sugars) or differential ones (generally pH and/or redox colour change indicators).

The method provides for the use of the agarized medium LAPTg, whose formulation consists solely in the presence of two different nitrogen sources, a sugar as a source of carbon, and yeast extract as a source of group B vitamins and growth factors. The absence of organic and inorganic salts and substances with selective action allows a flourishing growth of all the various species of probiotic bacteria belonging to the genera *Lactobacillus* and *Bifidobacterium.*

By adding a selective and/or differential agent to the LAPTg agar it is possible to perform differentiated counts of the probiotics present in a complex mixture. The selective agents (generally antibiotics and/or sugars) or differential ones (generally pH and/or redox colour change indicators) are selected case by case on the basis of the specific genotypic and phenotypic characteristics of the strains making up the mixture.

If the sample to be analyzed consists of a mixture of two or more strains of lactobacilli and bifidobacteria, it is advisable to accompany the selective count in LAPTg with a qualitative assessment of the strains making up the mixture using HHD medium. For further details, reference is made to the following scientific articles:

- Molecular Cloning a Laboratory Manual (Sambrook, Fritsch, Maniatis);
- Susceptibility of Lactobacillus spp. to antimicrobial agents. M. Danielsen A. Wind. 2002;
- Antibiotic Susceptibility of Lactobacillus and Bifidobacterium species from Human Gastrointestinal tract, S. Delgrado A.B. Florez B. Mayo, 2005;
- ISO 6887-1:2000
- Preparation of LAPTg medium: FONT DE VALDEZ, G, and coll. : Influence of the recovery medium on the viability of injured freeze-dried lactic acid bacteria Milchwissenschaft 40 (9) 518-520 (1985*).
- UNI EN ISO 6887-1:2000 "Buffered Peptone Water"
- A differential medium for the enumeration of homofermentative and heterofermentative lactic acid bacteria. LC. McDonald R.F. McFeeters, M.A. Daeschel and HP Felming. Applied and Environmental Microbiology. June 1987: 1382-1384.

Initials and abbreviations:

- concentration of live, viable cells = no. of cells/units (g or ml) able to grow in the culture medium and form distinct colonies (CFU/g or ml)
- CFU/g or ml = Colony Forming Unit, i.e. unit of measure of the concentration of live, viable cells
- MIC = Minimum Inhibitory Concentration

The method provides for the use of the agarized medium LAPTg, whose formulation consists solely in the presence of two different nitrogen sources, a sugar, as a source of carbon, and yeast extract as a source of group B vitamins and growth factors. The absence of organic and inorganic salts and substances with selective action allows a flourishing growth of all the various species of probiotic bacteria belonging to the genera *Lactobacillus* and *Bifidobacterium.* By adding a selective and/or differential agent to LAPTg agar it is possible to perform differentiated counts of the probiotics present in a complex mixture. The selective agents (generally antibiotics and/or sugars) or differential ones (generally pH and/or redox colour change indicators) are selected case by case on the basis of the specific genotypic and phenotypic characteristics of the strains making up the mixture. (see 8.2). If the sample to be analyzed consists of a mixture of two or more strains of lactobacilli and bifidobacteria, it is advisable to accompany the selective count in LAPTg with a qualitative assessment of the strains making up the mixture using HHD medium.

Materials and reagents:

LAPTg Medium, Basal Medium:

- Bacto Peptone (enzymatic hydrolysate of animal protein) 15 g
- Tryptone (pancreatic hydrolysate of casein) 10 g
- Yeast extract 10 g
- Tween 80 ml 1
- Agar g 15
- Distilled water q.s. to 900 ml

Note: the weights indicated above are understood as having an accuracy of $\pm$ 5%

Dissolve the components in the distilled water, except for the agar. Check the pH and correct if necessary to $6.55\pm0.05$, then add the agar and dissolve in a water bath. Dispense the medium while still warm into the Bibby beakers, and sterilize in an autoclave at 121°C for 15 minutes; after sterilization the pH should be $6.5\pm0.5$ at $25°C\pm1$.

Complete medium:

At the time of use, after dissolution (8.1), add one volume of a 10% glucose solution, sterilized by filtration, to the basal medium, so as to have a final glucose concentration of 10 g / litre.

HHD Medium:

| - | Fructose | 2.50 g |
|---|---|---|
| - | KH2PO4 | 2.50 g |
| - | Bacto Tryptone | 10.00 g |
| - | Soytone Peptone | 1.50 g |
| - | Casamino acids | 3.00 g |
| - | Yeast extract | 10.00 g |
| - | Tween 80 | 1.00 g |
| - | Bromocresolgreen | 20.00 ml |
| - | Agar | 20.00 g |
| - | Distilled water | 1000ml |

Final pH = 6.90 + 0.10.

Ethanol, Minisart single-use sterile 0.45μm syringe filters, diluents for reconstituting the samples: Reconstitution of liquid bacterial cultures and preparation of serial decimal dilutions - peptone saline solution

| | |
|---|---|
| - bacto peptone | 1.0 g |
| - sodium chloride (NaCl) | 8.5 g |
| - distilled water q.s to | 1000 ml |

Reconstitution of anhydrous (lyophilized) bacterial cultures and finished products with probiotics in free form (not microencapsulated).

Lyophilized) bacterial cultures not prepared in doses or units

Phosphate buffer pH 6.8

| | |
|---|---|
| - sodium chloride (NaCl) | 5.00 g |
| - $KH_2PO_4$ | 3.78 g |
| - $Na_2HPO_4$ | 4.77 g |
| - distilled water q.s. to | 1000 $\mu$m |

Sachets, capsules, pills, tablets, suppositories and undercaps of bottles:
Phosphate buffer pH 6.8

| | |
|---|---|
| - sodium chloride (NaCl) | g 5.00 |
| - $KH_2PO_4$ | g 3.78 |
| - $Na_2HPO_4$ | g 4.77 |
| - distilled water q.s. to | ml 1000 |

Dissolve the components in distilled water, heating if necessary. Check that the pH is 6.8±0.10. Dispense the diluents into Bibby beakers, sterilize in an autoclave at 121°C for 15 minutes, and store in darkness at a temperature of 4-5°C for no longer than one month.

Should the finished products contain oils and/or lipid substances it will be necessary to add 1% Tween 80 to the buffer pH 6.8 (5.5.2.1).

Anaerobic kit (AnaeroGen - Oxoid): chemically binds oxygen, producing $CO_2$; small 10ml non-sterile syringes also purchasable in pharmacies; 5%$_{fin}$ L-cysteine HCl solution: weigh out 5g of L-cysteine hydrochloride 1-hydrate (BDH 370553) and bring to 100ml with MQ water; then filter in a sterile falcon tube with a 0.45$\mu$m filter and store at +4°C±2 for up to 1 year (the solution will have to be added to the LAPTg medium so as to obtain a final concentration of 0.05%).

Procedure.

8.1 Dissolve the LAPTg medium (5.1) in a sufficient amount for the number of plates to be prepared (see note in paragraph 8.5.5), considering that for each plate about 12 ± 1 ml of medium is necessary. If it is planned to perform a count on the same dilutions of the sample not only in the LAPTg medium as such, but also in the same supplemented with one or more selective agents (N), consider the (no. of plates) multiplied by N. Leave the medium a thermostated water bath at a temperature of 45°C ± 0.5 for at least 3 hours;

8.2 List of selective agents and respective preparation

8.2.1 Antibiotics

➤ VANCOMICIN

| | CHLORAMPHENICOL |
|---|---|
| stock solution | 1mg/ml (sterilized by filtration 0.45$\mu$m) |
| diluent | water |
| usage concentration | 1 $\mu$g/ml |
| positive selection | obligate and facultative heterofermenter lactobacilli (e.g. *L. plantarum, L. rhamosus, L. casei, L. paracasei* etc.). |

(continued)

| | |
|---|---|
| negative selection | sensitive strains such as homofermenter lactobacilli (e.g. *L. acidophilus group*) and bifidobacteria. |

Storage: 1.5 ml aliquots at +4°C for 15 days or at -20°C for up to 4 months.

➢ CLINDAMYCIN + CIPROFLOXACIN

| | |
|---|---|
| stock solution | 10mg/ml (sterilized by filtration 0.45μm) |
| diluent | ethanol |
| usage concentration | 7 μg/ml |
| positive selection | strains with MIC > 4 μg/ml (e.g. LPC 00) |
| negative selection | strains with higher sensitivity (MIC < 2 μg/ml). |

Storage: 2 ml aliquots at -20°C for up to 4 months.

➢ CEFUROXIME

| | |
|---|---|
| stock solution | 1mg/ml clindamycin + 10mg/ml ciprofloxacin (sterilized by filtration 0.45μm) |
| diluent | water (in case of poor dissolution of the ciprofloxacin, add 1-2 drops of concentrated lactic acid). |
| usage concentration | 0.1 μg/ml clindamycin and 10 μg/ml ciprofloxacin |
| positive selection | *L. acidophilus group* |
| negative selection | *L. rhamosus, L. casei, L. paracasei, L. plantarum, L. reuteri, L. delbrueckii,* bifidobacteria, lattococci and *Streptococcus thermophilus.* |

Storage: 1.5 ml aliquots at -20°C for up to 6 months for ciprofloxacin (clindamycin n.d.).

➢ CEFOXITIN

| | |
|---|---|
| stock solution | 1 mg/ml (sterilized by filtration 0.45μm) |
| diluent | water |
| usage concentration | 0.1μg/ml |
| positive selection | strains with MIC > 1 (e.g. *B. longum* PCB 133) |
| negative selection | *Streptococcus thermophilus* (e.g. YO 8) and other strains with MIC ≤ 0.016 |

Storage: 1.5 ml aliquots at -20°C for up to 2 years.

➢ MUPIROCIN

| | |
|---|---|
| stock solution | 10 mg/ml (sterilized by filtration 0.45μm) |
| diluent | water |
| usage concentration | 100μg/ml |
| positive selection | strains with MIC > 256 (e.g. LGG, LPC 08, LC 01) |
| negative selection | LP 01, LP 02, *L. acidophilus* group and other sensitive strains with MIC < 24 |

Storage: 1.5 ml aliquots at -20°C for up to 2 years

➢ CIPROFLOXACIN (positive selection LP02 in admixture with LF10).

| | |
|---|---|
| stock solution | 10 mg/ml (sterilized by filtration 0.45μm) |
| diluent | water |
| usage concentration | 50μg/ml |
| positive selection | *Bifidobacteria* |
| negative selection | *Lactobacilli.* |

Storage: 1.5 ml aliquots at -20°C for up to 6 months

| | |
|---|---|
| stock solution | 10 mg/ml (sterilized by filtration 0.45μm) |

(continued)

| | |
|---|---|
| diluent | water (in case of poor dissolution of the ciprofloxacin, add 1-2 drops of concentrated lactic acid) |
| usage concentration | 5μg/ml |
| positive selection | *LP02* |
| negative selection | *LF10.* |

Storage: 1.5 ml aliquots at -20°C for up to 6 months

8.2.2 Other selective conditions.

8.3 If the sample to be analyzed consists of a mixture of two or more strains of lactobacilli and/or bifidobacteria, it is advisable to accompany the selective count in LAPTg with a qualitative assessment of the strains making up the mixture using HHD medium. Dissolve the aforesaid medium and leave it in a termostated bath as described for the LAPTg medium. Then distribute the medium in amounts of $12 \pm 1$ ml in Petri plates and allow to solidify;

8.4 If it is planned to use one or more selective agents, add the antibiotic solution or other selective agent necessary for discriminating the strains making up the sample to an aliquot (e.g. 100ml for about 8 plates) of the dissolved LAPTg medium, at the final concentration indicated, in a sterile bottle;

8.5 Prepare successive decimal dilutions of the sample (section la ISO 6887-1:2000 par. 9.2.)

8.5.1 Use a sterile pipette to transfer 1 ml of the primary dilution, or 1 ml directly from the sample culture if liquid, into a test tube containing 9 ml of sterile diluent;

8.5.2 do not introduce the pipette deeper than 1 cm into the initial suspension;

8.5.3 change the pipette after every dilution;

8.5.4 thoroughly homogenize the dilution with a mechanical shaker, vortexing the tube 3 times for a time of no less than 5 seconds, so as to obtain the dilution $10^{-2}$;

8.5.5 repeat these steps using the dilution $10^{-2}$ and dilute further until obtaining a concentration of microorganisms which, when cultured on a plate, give a significant number of colonies;

It should be noted that seeding 2 successive decimal dilutions (e.g.: $10^{-8}$, $10^{-9}$) should enable two contiguous dilutions containing a number of cells comprised from 10 to 300 to be found. If there is no clear idea as to the number of cells contained in the sample, it will be necessary to seed more than 2 successive decimal dilutions (e.g.: $10^{-5}$, $10^{-6}$, $10^{-7}$, $10^{-8}$, $10^{-9}$, $10^{-10}$) and then consider only the ones that give rise to a number of colonies comprised from 10 to 300.

8.6 distribute 1 ml, drawn from the dilutions of the sample judged to be appropriate (8.5.5), onto the Petri plates;

8.7 add the medium LAPTg on the first series of plates and LAPTg supplemented with the selective agent on the second series, in amounts of $12 \pm 1$ ml;

It should be noted that the time elapsing between the reconstitution of the sample and the moment at which the serial dilution comes into contact with the culture medium on the Petri plate (8.6) must not exceed 30 minutes

8.8 evenly mix the medium and sample, first with rotational movements, and then horizontal and vertical translational ones;

8.9 allow to solidify for 15-20 minutes;

8.10 in the case of samples consisting of a mixture of two or more strains of lactobacilli and bifidobacteria and/or for which it is recommended to use the HHD medium, add 100μl of the appropriate dilutions to the ready plates of HHD (8.3) and distribute the sample evenly with a spatula;

8.11 incubate the plates upside down at $37 \pm 1°C$ for 72h under anaerobic conditions (Gas-Pak + anaerobic kit). As to how to use the anaerobic system, follow the instructions included with the kit.

Calculation of the results: verify the presence of colonies by observing them under the lens of the plate viewer and count exclusively the plates containing a number of colonies comprised from 10 to 300. The result will be expressed as CFU, i.e. Colony Forming Units. Express the result using the following formula:

$$\frac{\Sigma C}{(n_1 + 0.1 \, n_2) \, d}$$

where:

$\Sigma C$ is the sum of the colonies counted on all the plates
$n_1$ is the number of plates counted in the first dilution
$n_2$ is the number of plates counted in the second dilution
$d$ is the dilution the first counts were obtained from

Example:

| | | |
|---|---|---|
| Plates of dilution $10^{-8}$ | : | 250 |
| Plates of dilution $10^{-9}$ | : | 23 |

Result:

$$\frac{250 + 23}{(1 + 0.1)\, 10^{-8}} = 248\ 10^{-8}\ CFU/g$$

Round off the result obtained to two significant digits. The results of the example shown will thus be 250 *$10^{-8}$ CFU/g* or *ml* in the case of liquid samples (for the detail of the expression of results based on the specific type. In the case of samples spread in HHD, visually examine the different morphologies of the cultured colonies which identify the various strains of lactobacilli and bifidobacteria present in admixture.

After performing the count and examining the plate-cultured colonies (step 9) one has: in the case of single-strain samples, the count obtained from the plates with LAPTg as such represents the total load of the probiotic load making up the sample.

In the case of samples consisting of a mixture of two or more strains of lactobacilli and bifidobacteria:

a) the count obtained from the series of plates prepared with the LAPTg medium as such represents the total load of the probiotic bacteria in the sample subjected to analysis;
b) the count obtained from the series of plates prepared with the LAPTg medium supplemented with the selective agent represents the load of the strain(s) that were capable of replicating in the presence of that specific substance added to the medium as a selective agent.
c) from the count regarding the individual probiotic strains, obtained with the selective medium, it is possible to derive the count of the remaining strain(s) that did not develop in the presence of the selective agent by calculating the difference with the total count (letter a) in LAPTg as such.
d) seeding in HHD medium makes it possible to visually discriminate the different strains present in the sample in admixture and selectively enumerated in LAPTg medium.

The Applicant tested the following raw materials with the method of the present invention in a number of tests using the probiotic bacterial strains indicated in Table 1 with the numbers 17, 22, 41, 60, 74, 98, 121, 145, 174 and 182 as toxicity markers.

Experimental data show that in many cases toxicity was unexpectedly found always to be present at various levels, along with a substantial % mortality.

**Aloe test:** mortality found equal to 5%.
**Citric Acid test:** mortality found equal to 2%.
**Arabinogalactan test:** mortality found equal to 6%.
**Raspberry flavouring test:** mortality found equal to 2%.
**Raspberry flavouring test:** mortality found equal to 26%.
**Blueberry test:** mortality found equal to 5%.
**Silicon dioxide test:** mortality found equal to 50%.
**Silicon dioxide test:** mortality found equal to 28%.
**Silicon dioxide test:** mortality found equal to 23%.

**Tara gum test:** mortality found equal to 14%.
**Vitamin B1, B2 and B6 test:** mortality found equal to 33%.
**Zeolite test:** mortality found equal to 2%.

**Claims**

1. A method for determining the toxicity of a food or pharmaceutical raw material which comprises a first step in which a food or pharmaceutical raw material is placed in contact with a pre-established bacterial load of a probiotic bacterial strain used as a toxicity marker and a second step of determining the reduction of said bacterial load due to the toxicity exerted by said food or pharmaceutical raw material toward said probiotic bacterial strain, wherein said first step comprises preparing:

   - a first test sample comprising the probiotic bacterial strain marker at a pre-established concentration, preferably at a concentration comprised from $1 \times 10^6$ to $1 \times 10^9$ CFU/g, the optimal culture substrate for the growth of said probiotic bacterial strain and the food or pharmaceutical raw material to be tested, and
   - a second test sample (internal reference) comprising the same probiotic bacterial strain of said first sample at the same pre-established concentration and the optimal culture substrate for the growth of said probiotic bacterial strain and wherein said second step comprises performing a bacterial count on said first and said second test samples to provide a count of the bacteria which survived in contact with the raw material and to express the % mortality induced by said raw material in the probiotic strain used as a marker, wherein the probiotic bacterial strain used as a toxicity marker is selected from .

Table 1

| No. | Name | Depositary institution | Deposit number | Deposit date | Depositor |
|---|---|---|---|---|---|
| 1 | *Lactobacillus casei* | CNCM I.P. | I-785 | 21.07.1988 | Anidral Srl |
| 2 | *Lactobacillus gasseri* | CNCM I.P. | I-786 | 21.07.1988 | Anidral Srl |
| 3 | *Lactobacillus crispatus* | CNCM I.P. | I-787 | 21.07.1988 | Anidral Srl |
| 4 | *Lactobacillus fermentum* | CNCM I.P. | I-788 | 21.07.1988 | Anidral Srl |
| 5 | *Lactobacillus fermentum* | CNCM I.P. | I-789 | 21.07.1988 | Anidral Srl |
| 6 | *Lactobacillus casei* ssp. *pseudoplantarum* | CNCM I.P. | I-790 | 21.07.1988 | Anidral Srl |
| 7 | *Streptococcus thermophilus* B39 | BCCM LMG | LMG P-18383 | 5.05.1998 | Anidral Srl |
| 8 | *Streptococcus thermophilus* T003 | BCCM LMG | LMG P-18384 | 5.05.1998 | Anidral Srl |
| 9 | *Lactobacillus pentosus* 9/1 ei | BCCM LMG | LMG P-21019 | 16.10.2001 | Mofin Srl |
| 10 | *Lactobacillus plantarum* 776 /1 bi | BCCM LMG | LMG P-21020 | 16.10.2001 | Mofin Srl |
| 11 | *Lactobacillus plantarum* 476LL 20 bi | BCCM LMG | LMG P-21021 | 16.10.2001 | Mofin Srl |
| 12 | *Lactobacillus plantarum* PR ci | BCCM LMG | LMG P-21022 | 16.10.2001 | Mofin Srl |
| 13 | *Lactobacillus plantarum* 776 /2 hi | BCCM LMG | LMG P-21023 | 16.10.2001 | Mofin Srl |
| 14 | *Lactobacillus casei* ssp. *paracasei* 181A/3 aiai | BCCM LMG | LMG P-21380 | 31.01.2002 | Anidral Srl |

(continued)

| No. | Name | Depositary institution | Deposit number | Deposit date | Depositor |
|---|---|---|---|---|---|
| 15 | *Lactobacillus* belonging to the *acidophilus* group 192A/1 aiai | BCCM LMG | LMG P-21381 | 31.01.2002 | Anidral Srl |
| 16 | *Bifidobacterium longum* 175A/1 aiai | BCCM LMG | LMG P-21382 | 31.01.2002 | Anidral Srl |
| 17 | *Bifidobacterium breve* 195A/1 aici | BCCM LMG | LMG P-21383 | 31.01.2002 | Anidral Srl |
| 18 | *Bifidobacterium lactis* 32A/3 aiai | BCCM LMG | LMG P-21384 | 31.01.2002 | Anidral Srl |
| 19 | *Lactobacillus plantarum* 501/2 gi | BCCM LMG | LMG P-21385 | 31.01.2002 | Mofin Srl |
| 20 | *Lactococcus lactis* ssp. *lactis* 501/4 ci | BCCM LMG | LMG P-21388 | 31.01.2002 | Mofin Srl |
| 21 | *Lactococcus lactis* ssp. *lactis* 501/4 hi | BCCM LMG | LMG P-21387 | 15.03.2002 | Mofin Srl |
| 22 | *Lactococcus lactis* ssp. *lactis* 501/4 ci | BCCM LMG | LMG P-21388 | 31.01.2002 | Mofin Srl |
| 23 | *Lactobacillus plantarum* 501/4 li | BCCM LMG | LMG P-21389 | 15.03.2002 | Mofin Srl |
| 24 | *Lactobacillus acidophilus* | BCCM LMG | LMG P-26144 | 03.11.2010 | Probiotical SpA |
| 25 | *Lactobacillus paracasei* ssp. *paracasei* | BCCM LMG | LMG P-26143 | 03.11.2010 | Probiotical SpA |
| 26 | *Streptococcus thermophilus* | DSMZ | DSM 16506 | 18.06.2004 | Anidral Srl |
| 27 | *Streptococcus thermophilus* | DSMZ | DSM 16507 | 18.06.2004 | Anidral Srl |
| 28 | *Streptococcus thermophilus* | DSMZ | DSM 16590 | 20.07.2004 | Anidral Srl |
| 29 | *Streptococcus thermophilus* | DSMZ | DSM 16591 | 20.07.2004 | Anidral Srl |
| 30 | *Streptococcus thermophilus* | DSMZ | DSM 16592 | 20.07.2004 | Anidral Srl |
| 31 | *Streptococcus thermophilus* | DSMZ | DSM 16593 | 20.07.2004 | Anidral Srl |
| 32 = 56 | *Bifidobacterium adolescentis* | DSMZ | DSM 16594 | 21.07.2004 | Anidral Srl |
| 33 | *Bifidobacterium adolescentis* | DSMZ | DSM 16595 | 21.07.2004 | Anidral Srl |
| 34 | *Bifidobacterium breve* | DSMZ | DSM 16596 | 21.07.2004 | Anidral Srl |
| 35 | *Bifidobacterium pseudocatenulatum* | DSMZ | DSM 16597 | 21.07.2004 | Anidral Srl |
| 36 | *Bifidobacterium pseudocatenulatum* | DSMZ | DSM 16598 | 21.07.2004 | Anidral Srl |

(continued)

| No. | Name | Depositary institution | Deposit number | Deposit date | Depositor |
|---|---|---|---|---|---|
| 37 | *Bifidobacterium longum* | DSMZ | DSM 16603 | 20.07.2004 | Anidral Srl |
| 38 | *Bifidobacterium breve* | DSMZ | DSM 16604 | 20.07.2004 | Anidral Srl |
| 39 | *Lactobacillus casei* ssp. *rhamnosus* | DSMZ | DSM 16605 | 20.07.2004 | Anidral Srl |
| 40 | *Lactobacillus delbrueckii* ssp. *bulgaricus* | DSMZ | DSM 16606 | 20.07.2004 | Anidral Srl |
| 41 | *Lactobacillus delbrueckii* ssp. *bulgaricus* | DSMZ | DSM 16607 | 20.07.2004 | Anidral Srl |
| 42 | *Staphylococcus xylosus* | DSMZ | DSM 17102 | 01.02.2005 | Anidral Srl |
| 43 = 57 | *Bifidobacterium adolescentis* | DSMZ | DSM 17103 | 01.02.2005 | Anidral Srl |
| 44 | *Lactobacillus plantarum* | DSMZ | DSM 17104 | 01.02.2005 | Anidral Srl |
| 45 | *Streptococcus thermophilus* | DSMZ | DSM 17843 | 21.12.2005 | Anidral Srl |
| 46 | *Streptococcus thermophilus* | DSMZ | DSM 17844 | 21.12.2005 | Anidral Srl |
| 47 | *Streptococcus thermophilus* | DSMZ | DSM 17845 | 21.12.2005 | Anidral Srl |
| 48 | *Lactobacillus fermentum* | DSMZ | DSM 18295 | 24.05.2006 | Anidral Srl |
| 49 | *Lactobacillus fermentum* | DSMZ | DSM 18296 | 24.05.2006 | Anidral Srl |
| 50 | *Lactobacillus fermentum* | DSMZ | DSM 18297 | 24.05.2006 | Anidral Srl |
| 51 | *Lactobacillus fermentum* | DSMZ | DSM 18298 | 24.05.2006 | Anidral Srl |
| 52 | *Lactobacillus gasseri* | DSMZ | DSM 18299 | 24.05.2006 | Anidral Srl |
| 53 | *Lactobacillus gasseri* | DSMZ | DSM 18300 | 24.05.2006 | Anidral Srl |
| 54 | *Lactobacillus gasseri* | DSMZ | DSM 18301 | 24.05.2006 | Anidral Srl |
| 55 | *Lactobacillus gasseri* | DSMZ | DSM 18302 | 24.05.2006 | Anidral Srl |
| 56 = 32 | *Bifidobacterium adolescentis* EI-3 ***Bifidobacterium catenulatum sp./ pseudocatenul atum* EI-3I, ID 09-255** | DSMZ | DSM 18350 | 15.06.2006 | Anidral Srl |
| 57 = 43 | *Bifidobacterium adolescentis* EI-15 | DSMZ | DSM 18351 | 15.06.2006 | Anidral Srl |

(continued)

| No. | Name | Depositary institution | Deposit number | Deposit date | Depositor |
|-----|------|------------------------|----------------|--------------|-----------|
| 58 | *Bifidobacterium adolescentis* EI-18<br>***Bifidobacterium animalis subsp. lactis* EI-18, ID 09-256** | DSMZ | DSM 18352 | 15.06.2006 | Anidral Srl |
| 59 | *Bifidobacterium catenulatum* EI-20 | DSMZ | DSM 18353 | 15.06.2006 | Anidral Srl |
| 60 | *Streptococcus thermophilus* FRai | DSMZ | DSM 18613 | 13.09.2006 | Mofin Srl |
| 61 | *Streptococcus thermophilus* LB2bi | DSMZ | DSM 18614 | 13.09.2006 | Mofin Srl |
| 62 | *Streptococcus thermophilus* LRci | DSMZ | DSM 18615 | 13.09.2006 | Mofin Srl |
| 63 | *Streptococcus thermophilus* FP4 | DSMZ | DSM 18616 | 13.09.2006 | Mofin Srl |
| 64 | *Streptococcus thermophilus* ZZ5F8 | DSMZ | DSM 18617 | 13.09.2006 | Mofin Srl |
| 65 | *Streptococcus thermophilus* TEO4 | DSMZ | DSM 18618 | 13.09.2006 | Mofin Srl |
| 66 | *Streptococcus thermophilus* S1ci | DSMZ | DSM 18619 | 13.09.2006 | Mofin Srl |
| 67 | *Streptococcus thermophilus* 641bi | DSMZ | DSM 18620 | 13.09.2006 | Mofin Srl |
| 68 | *Streptococcus thermophilus* 277A/1ai | DSMZ | DSM 18621 | 13.09.2006 | Mofin Srl |
| 69 | *Streptococcus thermophilus* 277A/2ai | DSMZ | DSM 18622 | 13.09.2006 | Mofin Srl |
| 70 | *Streptococcus thermophilus* IDC11 | DSMZ | DSM 18623 | 13.09.2006 | Mofin Srl |
| 71 | *Streptococcus thermophilus* ML3di | DSMZ | DSM 18624 | 13.09.2006 | Mofin Srl |
| 72 | *Streptococcus thermophilus* TEO3 | DSMZ | DSM 18625 | 13.09.2006 | Mofin Srl |
| 73 | *Streptococcus thermophilus* G62 | DSMZ | DSM 19057 | 21.02.2007 | Mofin Srl |
| 74 | *Streptococcus thermophilus* G1192 | DSMZ | DSM 19058 | 21.02.2007 | Mofin Srl |
| 75 | *Streptococcus thermophilus* GB18 | DSMZ | DSM 19059 | 21.02.2007 | Mofin Srl |
| 76 | *Streptococcus thermophilus* CCR21 | DSMZ | DSM 19060 | 21.02.2007 | Mofin Srl |
| 77 | *Streptococcus thermophilus* G92 | DSMZ | DSM 19061 | 21.02.2007 | Mofin Srl |
| 78 | *Streptococcus thermophilus* G69 | DSMZ | DSM 19062 | 21.02.2007 | Mofin Srl |

(continued)

| No. | Name | Depositary institution | Deposit number | Deposit date | Depositor |
|---|---|---|---|---|---|
| 79 | *Streptococcus thermophilus* | DSMZ | DSM 19063 | 21.02.2007 | Anidral Srl |
| 80 | *Streptococcus thermophilus* | DSMZ | DSM 19064 | 21.02.2007 | Anidral Srl |
| 81 | *Streptococcus thermophilus* | DSMZ | DSM 19065 | 21.02.2007 | Anidral Srl |
| 82 | *Streptococcus thermophilus* | DSMZ | DSM 19066 | 21.02.2007 | Anidral Srl |
| 83 | *Weissella* ssp. WSP 01 | DSMZ | DSM 19067 | 21.02.2007 | Anidral Srl |
| 84 | *Weissella* ssp. WSP 02 | DSMZ | DSM 19068 | 21.02.2007 | Anidral Srl |
| 85 | *Lactobacillus* ssp. WSP 03 | DSMZ | DSM 19069 | 21.02.2007 | Anidral Srl |
| 86 | *Lactobacillus plantarum* LP 09 | DSMZ | DSM 19070 | 21.02.2007 | Anidral Srl |
| 87 | *Lactobacillus plantarum* LP 10 | DSMZ | DSM 19071 | 21.02.2007 | Anidral Srl |
| 88 | *Lactococcus lactis* | DSMZ | DSM 19072 | 21.02.2007 | Anidral Srl |
| 89 | *Lactobacillus fermentum* | DSMZ | DSM 19187 | 20.03.2007 | Anidral Srl |
| 90 | *Lactobacillus fermentum* | DSMZ | DSM 19188 | 20.03.2007 | Anidral Srl |
| 91 | *Lactobacillus casei* ssp. *rhamnosus* | DSMZ | DSM 19739 | 27.09.2007 | Anidral Srl |
| 92 | *Bifidobacterium bifidum* | DSMZ | DSM 19818 | 30.10.2007 | Anidral Srl |
| 93 | *Lactobacillus delbrueckii* subsp. *bulgaricus* LD 01 | DSMZ | DSM 19948 | 28.11.2007 | Anidral Srl |
| 94 | *Lactobacillus delbrueckii* subsp. *bulgaricus* LD 02 | DSMZ | DSM 19949 | 28.11.2007 | Anidral Srl |
| 95 | *Lactobacillus delbrueckii* subsp. *bulgaricus* LD 03 | DSMZ | DSM 19950 | 28.11.2007 | Anidral Srl |
| 96 | *Lactobacillus delbrueckii* subsp. *bulgaricus* LD 04 | DSMZ | DSM 19951 | 28.11.2007 | Anidral Srl |
| 97 | *Lactobacillus delbrueckii* subsp. *bulgaricus* LD 05 | DSMZ | DSM 19952 | 28.11.2007 | Anidral Srl |
| 98 | *Bifidobacterium pseudocatenulatum* | DSMZ | DSM 21444 | 13.05.2008 | Probiotical SpA |
| 99 | *Lactobacillus acidophilus* | DSMZ | DSM 21717 | 06.08.2008 | Probiotical SpA |
| 100 | *Lactobacillus paracasei* | DSMZ | DSM 21718 | 06.08.2008 | Probiotical SpA |

(continued)

| No. | Name | Depositary institution | Deposit number | Deposit date | Depositor |
|-----|------|------------------------|----------------|--------------|-----------|
| 101 | *Lactobacillus pentosus* | DSMZ | DSM 21980 | 14.11.2008 | Probiotical SpA |
| 102 | *Lactobacillus rahmnosus* | DSMZ | DSM 21981 | 14.11.2008 | Probiotical SpA |
| 103 | *Lactobacillus delbrueckii* ssp. *delbrueckii* | DSMZ | DSM 22106 | 10.12.2008 | Probiotical SpA |
| 104 | *Lactobacillus plantarum* | DSMZ | DSM 22107 | 10.12.2008 | Probiotical SpA |
| 105 | *Lactobacillus salivarius* | DSMZ | DSM 22775 | 23.07.2009 | Probiotical SpA |
| 106 | *Lactobacillus salivarius* | DSMZ | DSM 22776 | 23.07.2009 | Probiotical SpA |
| 107 | *Bifidobacterium bifidum* | DSMZ | DSM 22892 | 28.08.2009 | Probiotical SpA |
| 108 | *Bifidobacterium bifidum* | DSMZ | DSM 22893 | 28.08.2009 | Probiotical SpA |
| 109 | *Bifidobacterium bifidum* | DSMZ | DSM 22894 | 28.08.2009 | Probiotical SpA |
| 110 | *Bifidobacterium lactis* | DSMZ | DSM 23032 | 13.10.2009 | Probiotical SpA |
| 111 | *Lactobacillus acidophilus* | DSMZ | DSM 23033 | 13.10.2009 | Probiotical SpA |
| 112 | *Lactobacillus brevis* | DSMZ | DSM 23034 | 13.10.2009 | Probiotical SpA |
| 113 | *Bifidobacterium animalis* ssp. *lactis* | DSMZ | DSM 23224 | 12.01.2010 | Probiotical SpA |
| 114 | *Bifidobacterium longum* | DSMZ | DSM 23233 | 12.01.2010 | Probiotical SpA |
| 115 | *Bifidobacterium longum* | DSMZ | DSM 23234 | 12.01.2010 | Probiotical SpA |
| 116 | *Bifidobacterium bifidum* | DSMZ | DSM 23731 | 29.06.2010 | Probiotical SpA |
| 117 | *Bifidobacterium breve* | DSMZ | DSM 23732 | 29.06.2010 | Probiotical SpA |
| 118 | *Bifidobacterium lactis* | DSMZ | DSM 23733 | 29.06.2010 | Probiotical SpA |
| 119 | *Lactobacillus reuteri* | DSMZ | DSM 23877 | 05.08.2010 | Probiotical SpA |
| 120 | *Lactobacillus reuteri* | DSMZ | DSM 23878 | 05.08.2010 | Probiotical SpA |
| 121 | *Lactobacillus reuteri* | DSMZ | DSM 23879 | 05.08.2010 | Probiotical SpA |
| 122 | *Lactobacillus reuteri* | DSMZ | DSM 23880 | 05.08.2010 | Probiotical SpA |

(continued)

| No. | Name | Depositary institution | Deposit number | Deposit date | Depositor |
|---|---|---|---|---|---|
| 123 | *Lactobacillus paracasei* ssp. *paracasei* | DSMZ | DSM 24243 | 23.11.2010 | Probiotical SpA |
| 124 | *Lactobacillus acidophilus* | DSMZ | DSM 24303 | 23.11.2010 | Probiotical SpA |
| 125 | *Bifidobacterium bifidum* | DSMZ | DSM 24437 | 04.01.2011 | Probiotical SpA |
| 126 | *Lactobacillus crispatus* | DSMZ | DSM 24438 | 04.01.2011 | Probiotical SpA |
| 127 | *Lactobacillus crispatus* | DSMZ | DSM 24439 | 04.01.2011 | Probiotical SpA |
| 128 | *Lactobacillus paracasei* | DSMZ | DSM 24440 | 04.01.2011 | Probiotical SpA |
| 129 | *Lactobacillus salivarius* | DSMZ | DSM 24441 | 04.01.2011 | Probiotical SpA |
| 130 | *Lactobacillus gasseri* | DSMZ | DSM 24512 | 25.01.2011 | Probiotical SpA |
| 131 | *Lactobacillus acidophilus* | DSMZ | DSM 24513 | 25.01.2011 | Probiotical SpA |
| 132 | *Lactobacillus salivarius* | DSMZ | DSM 24618 | 02.03.2011 | Probiotical SpA |
| 133 | *Lactobacillus crispatus* | DSMZ | DSM 24619 | 02.03.2011 | Probiotical SpA |
| 134 | *Lactobacillus crispatus* | DSMZ | DSM 24620 | 02.03.2011 | Probiotical SpA |
| 135 | *Lacotbacillus acidophilus* | DSMZ | DSM 24621 | 02.03.2011 | Probiotical SpA |
| 136 | *Lactobacillus gasseri* | DSMZ | DSM 24622 | 02.03.2011 | Probiotical SpA |
| 137 | *Lactobacillus paracasei* | DSMZ | DSM 24623 | 02.03.2011 | Probiotical SpA |
| 138 | *Bifidobacterium infantis* | DSMZ | DSM 24687 | 29.03.2011 | Probiotical SpA |
| 139 | *Bifidobacterium bifidum* | DSMZ | DSM 24688 | 29.03.2011 | Probiotical SpA |
| 140 | *Bifidobacterium longum* | DSMZ | DSM 24689 | 29.03.2011 | Probiotical SpA |
| 141 | *Bifidobacterium lactis* | DSMZ | DSM 24690 | 29.03.2011 | Probiotical SpA |
| 142 | *Bifidobacterium longum* | DSMZ | DSM 24691 | 29.03.2011 | Probiotical SpA |
| 143 | *Bifidobacterium breve* | DSMZ | DSM 24706 | 07.04.2011 | Probiotical SpA |
| 144 | *Bifidobacterium breve* | DSMZ | DSM 24707 | 07.04.2011 | Probiotical SpA |

(continued)

| No. | Name | Depositary institution | Deposit number | Deposit date | Depositor |
|-----|------|------------------------|----------------|--------------|-----------|
| 145 | *Bifidobacterium breve* | DSMZ | DSM 24708 | 07.04.2011 | Probiotical SpA |
| 146 | *Bifidobacterium longum* | DSMZ | DSM 24709 | 07.04.2011 | Probiotical SpA |
| 147 | *Lactobacillus salivarius* | DSMZ | DSM 25138 | 02.09.2011 | Probiotical SpA |
| 148 | *Lactobacillus reuteri* | DSMZ | DSM 25139 | 02.09.2011 | Probiotical SpA |
| 149 | *Lactobacillus reuteri* | DSMZ | DSM 25140 | 02.09.2011 | Probiotical SpA |
| 150 | *Lactobacillus reuteri* | DSMZ | DSM 25141 | 02.09.2011 | Probiotical SpA |
| 151 | *Streptococcus thermophilus* | DSMZ | DSM 25246 | 19.09.2011 | Probiotical SpA |
| 152 | *Streptococcus thermophilus* | DSMZ | DSM 25247 | 19.09.2011 | Probiotical SpA |
| 153 | *Streptococcus thermophilus* | DSMZ | DSM 25282 | 20.10.2011 | Probiotical SpA |
| 154 | *Lactobacillus salivarius* | DSMZ | DSM 25545 | 12.01.2012 | Probiotical SpA |
| 155 | *Bifidobacterium longum* | DSMZ | DSM 25669 | 16.02.2012 | Probiotical SpA |
| 156 | *Bifidobacterium longum* | DSMZ | DSM 25670 | 16.02.2012 | Probiotical SpA |
| 157 | *Bifidobacterium longum* | DSMZ | DSM 25671 | 16.02.2012 | Probiotical SpA |
| 158 | *Bifidobacterium longum* | DSMZ | DSM 25672 | 16.02.2012 | Probiotical SpA |
| 159 | *Bifidobacterium longum* | DSMZ | DSM 25673 | 16.02.2012 | Probiotical SpA |
| 160 | *Bifidobacterium longum* | DSMZ | DSM 25674 | 16.02.2012 | Probiotical SpA |
| 161 | *Bifidobacterium longum* | DSMZ | DSM 25675 | 16.02.2012 | Probiotical SpA |
| 162 | *Bifidobacterium longum* | DSMZ | DSM 25676 | 16.02.2012 | Probiotical SpA |
| 163 | *Bifidobacterium longum* | DSMZ | DSM 25677 | 16.02.2012 | Probiotical SpA |
| 164 | *Bifidobacterium longum* | DSMZ | DSM 25678 | 16.02.2012 | Probiotical SpA |
| 165 | *Bifidobacterium longum* | DSMZ | DSM 25679 | 16.02.2012 | Probiotical SpA |
| 166 | *Lactobacillus johnsonii* | DSMZ | DSM 25680 | 16.02.2012 | Probiotical SpA |

(continued)

| No. | Name | Depositary institution | Deposit number | Deposit date | Depositor |
|---|---|---|---|---|---|
| 167 | *Lactobacillus rhamnosus* | DSMZ | DSM 25681 | 16.02.2012 | Probiotical SpA |
| 168 | *Lactobacillus rhamnosus* | DSMZ | DSM 25682 | 16.02.2012 | Probiotical SpA |
| 169 | *Lactobacillus reuteri* | DSMZ | DSM 25683 | 16.02.2012 | Probiotical SpA |
| 170 | *Lactobacillus reuteri* | DSMZ | DSM 25684 | 16.02.2012 | Probiotical SpA |
| 171 | *Lactobacillus reuteri* | DSMZ | DSM 25685 | 16.02.2012 | Probiotical SpA |
| 172 | *Bifidobacterium longum* | DSMZ | DSM 25708 | 24.02.2012 | Probiotical SpA |
| 173 | *Bifidobacterium infantis* | DSMZ | DSM 25709 | 24.02.2012 | Probiotical SpA |
| 174 | *Lactobacillus plantarum* | DSMZ | DSM 25710 | 24.02.2012 | Probiotical SpA |
| 175 | *Bifidobacterium longum* | DSMZ | DSM 25717 | 01.03.2012 | Probiotical SpA |
| 176 | *Bifidobacterium longum* | DSMZ | DSM 25718 | 01.03.2012 | Probiotical SpA |
| 177 | *Lactobacillus salivarius* | DSMZ | DSM 26036 | 06.06.2012 | Probiotical SpA |
| 178 | *Lactobacillus salivarius* | DSMZ | DSM 26037 | 06.06.2012 | Probiotical SpA |
| 179 | *Lactobacillus pentosus* | DSMZ | DSM 26038 | 06.06.2012 | Probiotical SpA |
| 180 | *Bifidobacterium pseudolongum* ssp. *globosum* | DSMZ | DSM 26456 | 02.10.2012 | Probiotical SpA |
| 181 | *Lactobacillus fermentum* | DSMZ | DSM 26955 | 01.03.2013 | Probiotical SpA |
| 182 | *Lactobacillus fermentum* | DSMZ | DSM 26956 | 01.03.2013 | Probiotical SpA |
| 183 | *Lactobacillus casei* | DSMZ | DSM 27537 | 24.07.2013 | Probiotical SpA |
| 184 | *Lactobacillus crispatus* | DSMZ | DSM 27538 | 24.07.2013 | Probiotical SpA |
| 185 | *Lactobacillus jensenii* | DSMZ | DSM 27539 | 24.07.2013 | Probiotical SpA |

**2.** The method according to claim 1, wherein the probiotic bacterial strain used as a toxicity marker is selected from.

| 9 | *Lactobacillus pentosus* 9/1 ei | BCCM LMG | LMG P-21019 | 16.10.2001 | Mofin Srl |
|---|---|---|---|---|---|
| 15 | *Lactobacillus* belonging to the *acidophilus* group 192A/1 aiai | BCCM LMG | LMG P-21381 | 31.01.2002 | Anidral Srl |
| 17 | *Bifidobacterium breve* 195A/1 aici | BCCM LMG | LMG P-21383 | 31.01.2002 | Anidral Srl |
| 18 | *Bifidobacterium lactis* 32A/3 aiai | BCCM LMG | LMG P-21384 | 31.01.2002 | Anidral Srl |
| 22 | *Lactococcus lactis* ssp. *lactis* 501/4 ci | BCCM LMG | LMG P-21388 | 31.01.2002 | Mofin Srl |
| 33 | *Bifidobacterium adolescentis* | DSMZ | DSM 16595 | 21.07.2004 | Anidral Srl |
| 39 | *Lactobacillus casei* ssp. *rhamnosus* | DSMZ | DSM 16605 | 20.07.2004 | Anidral Srl |
| 41 | *Lactobacillus delbrueckii* ssp. *bulgaricus* | DSMZ | DSM 16607 | 20.07.2004 | Anidral Srl |
| 46 | *Streptococcus thermophilus* | DSMZ | DSM 17844 | 21.12.2005 | Anidral Srl |
| 54 | *Lactobacillus gasseri* | DSMZ | DSM 18301 | 24.05.2006 | Anidral Srl |
| 59 | *Bifidobacterium catenulatum* EI-20 | DSMZ | DSM 18353 | 15.06.2006 | Anidral Srl |
| 60 | *Streptococcus thermophilus* FRai | DSMZ | DSM 18613 | 13.09.2006 | Mofin Srl |
| 73 | *Streptococcus thermophilus* G62 | DSMZ | DSM 19057 | 21.02.2007 | Mofin Srl |
| 74 | *Streptococcus thermophilus* G1192 | DSMZ | DSM 19058 | 21.02.2007 | Mofin Srl |
| 84 | *Weissella* ssp. WSP 02 | DSMZ | DSM 19068 | 21.02.2007 | Anidral Srl |
| 95 | *Lactobacillus delbrueckii* subsp. *bulgaricus* LD 03 | DSMZ | DSM 19950 | 28.11.2007 | Anidral Srl |
| 98 | *Bifidobacterium pseudocatenulatum* | DSMZ | DSM 21444 | 13.05.2008 | Probiotical SpA |
| 101 | *Lactobacillus pentosus* | DSMZ | DSM 21980 | 14.11.2008 | Probiotical SpA |
| 105 | *Lactobacillus salivarius* | DSMZ | DSM 22775 | 23.07.2009 | Probiotical SpA |
| 116 | *Bifidobacterium bifidum* | DSMZ | DSM 23731 | 29.06.2010 | Probiotical SpA |

(continued)

| 121 | *Lactobacillus reuteri* | DSMZ | DSM 23879 | 05.08.2010 | Probiotical SpA |
|---|---|---|---|---|---|
| 130 | *Lactobacillus gasseri* | DSMZ | DSM 24512 | 25.01.2011 | Probiotical SpA |
| 138 | *Bifidobacterium infantis* | DSMZ | DSM 24687 | 29.03.2011 | Probiotical SpA |
| 143 | *Bifidobacterium breve* | DSMZ | DSM 24706 | 07.04.2011 | Probiotical SpA |
| 145 | *Bifidobacterium breve* | DSMZ | DSM 24708 | 07.04.2011 | Probiotical SpA |
| 169 | *Lactobacillus reuteri* | DSMZ | DSM 25683 | 16.02.2012 | Probiotical SpA |
| 174 | *Lactobacillus plantarum* | DSMZ | DSM 25710 | 24.02.2012 | Probiotical SpA |
| 182 | *Lactobacillus fermentum* | DSMZ | DSM 26956 | 01.03.2013 | Probiotical SpA |
| 185 | *Lactobacillus jensenii* | DSMZ | DSM 27539 | 24.07.2013 | Probiotical SpA |

3. The method according to claim 1 or 2, wherein said bacterial count is performed with a method comprising a step of re-suspending the initial sample, a step of making serial dilutions in a suitable diluent, a step of plating in an agarized medium and a step of counting the colonies after incubation under optimal conditions.

4. The method according to one of the preceding claims, wherein the ratio between the bacterial count (number of cells counted on the plate) of said first test sample and the bacterial count (number of cells counted on the plate) of said second test sample (internal reference) is determined.

5. The method according to one of the preceding claims, wherein the probiotic bacterial strains used as a marker of toxicity toward probiotic bacteria are selected from lactobacilli and bifidobacteria.

6. The method according to any one of the preceding claims, wherein said food or pharmaceutical raw material is selected from the group comprising flavourings, extracts, co-formulants of organic and/or inorganic origin, vitamins, proteins, amino acids, peptones, natural and/or synthetic polymers.

7. A method for producing food products or dietary supplements or medical devices or pharmaceutical products comprising raw materials that are not toxic toward the selected probiotic bacteria as defined in claim 1, said method comprising the method for determining the toxicity of a food or pharmaceutical raw material according to one or more of the preceding claims 1-6.

**Patentansprüche**

1. Verfahren zur Bestimmung der Toxizität eines Grundstoffs für ein Nahrungsmittel oder Arzneimittel, umfassend einen ersten Schritt, in welchem ein Grundstoff für ein Nahrungsmittel oder Arzneimittel mit einer vorbestimmten Bakterienlast eines als Toxizitätsmarker verwendeten probiotischen Bakterienstamms in Kontakt gebracht wird, und einen zweiten Schritt zur Bestimmung der Verringerung dieser Bakterienlast aufgrund der durch den Grundstoff für ein Nahrungsmittel oder Arzneimittel ausgeübten Toxizität gegenüber dem probiotischen Bakterienstamm, wobei der erste Schritt die Herstellung umfasst von:

- einer ersten Testprobe, umfassend den probiotischen Bakterienstammmarker in vorbestimmter Konzentration, vorzugsweise einer Konzentration von $1 \times 10^6$ bis $1 \times 10^9$ KBE/g, das optimale Kultursubstrat für das Wachstum des probiotischen Bakterienstamms und den zu untersuchenden Grundstoff für ein Nahrungsmittel oder Arzneimittel, und

- einer zweiten Testprobe (interne Referenz) mit dem gleichen probiotischen Bakterienstamm der ersten Probe bei gleicher vorbestimmter Konzentration und dem optimalen Kultursubstrat für das Wachstum des probiotischen Bakterienstamms,

und wobei der zweite Schritt die Durchführung einer Zählung der Bakterien in der ersten und der zweiten Testprobe umfasst, um eine Zahl der Bakterien anzugeben, die den Kontakt mit dem Grundstoff überlebt haben, und um die durch den Grundstoff ausgelöste prozentuelle Mortalität in dem als Marker verwendeten probiotischen Stamm darzustellen, wobei der als Toxizitätsmarker verwendete probiotische Bakterienstamm ausgewählt wird aus Tabelle 1:

| Nr. | Name | Hinterlegungsstelle | Hinterlegungsnummer | Hinterlegungsdatum | Hinterleger |
|---|---|---|---|---|---|
| 1 | Lactobacillus casei | CNCM I.P. | I-785 | 21.07.1988 | Anidral Srl |
| 2 | Lactobacillus gasseri | CNCM I.P. | I-786 | 21.07.1988 | Anidral Srl |
| 3 | Lactobacillus crispatus | CNCM I.P. | I-787 | 21.07.1988 | Anidral Srl |
| 4 | Lactobacillus fermentum | CNCM I.P. | I-788 | 21.07.1988 | Anidral Srl |
| 5 | Lactobacillus fermentum | CNCM I.P. | I-789 | 21.07.1988 | Anidral Srl |
| 6 | Lactobacillus casei ssp. pseudoplantarum | CNCM I.P. | I-790 | 21.07.1988 | Anidral Srl |
| 7 | Streptococcus thermophilus B39 | BCCM LMG | LMG P-18383 | 5.05.1998 | Anidral Srl |
| 8 | Streptococcus thermophilus T003 | BCCM LMG | LMG P-18384 | 5.05.1998 | Anidral Srl |
| 9 | Lactobacillus pentosus 9/1 ei | BCCM LMG | LMG P-21019 | 16.10.2001 | Mofin Srl |
| 10 | Lactobacillus plantarum 776 /1 bi | BCCM LMG | LMG P-21020 | 16.10.2001 | Mofin Srl |
| 11 | Lactobacillus plantarum 476LL 20 bi | BCCM LMG | LMG P-21021 | 16.10.2001 | Mofin Srl |
| 12 | Lactobacillus plantarum PR ci | BCCM LMG | LMG P-21022 | 16.10.2001 | Mofin Srl |
| 13 | Lactobacillus plantarum 776 /2 hi | BCCM LMG | LMG P-21023 | 16.10.2001 | Mofin Srl |
| 14 | Lactobacillus casei ssp. paracasei 181A/3 aiai | BCCM LMG | LMG P-21380 | 31.01.2002 | Anidral Srl |
| 15 | Lactobacillus aus der acidophilus Gruppe 192A/1 aiai | BCCM LMG | LMG P-21381 | 31.01.2002 | Anidral Srl |
| 16 | Bifidobacterium longum 175A/1 aiai | BCCM LMG | LMG P-21382 | 31.01.2002 | Anidral Srl |
| 17 | Bifidobacterium breve 195A/1 aici | BCCM LMG | LMG P-21383 | 31.01.2002 | Anidral Srl |
| 18 | Bifidobacterium lactis 32A/3 aiai | BCCM LMG | LMG P-21384 | 31.01.2002 | Anidral Srl |
| 19 | Lactobacillus plantarum 501/2 gi | BCCM LMG | LMG P-21385 | 31.01.2002 | Mofin Srl |
| 20 | Lactococcus lactis ssp. lactis 501/4 ci | BCCM LMG | LMG P-21388 | 31.01.2002 | Mofin Srl |
| 21 | Lactococcus lactis ssp. lactis 501/4 hi | BCCM LMG | LMG P-21387 | 15.03.2002 | Mofin Srl |
| 22 | Lactococcus lactis ssp. lactis 501/4 ci | BCCM LMG | LMG P-21388 | 31.01.2002 | Mofin Srl |
| 23 | Lactobacillus plantarum 501/4 li | BCCM LMG | LMG P-21389 | 15.03.2002 | Mofin Srl |
| 24 | Lactobacillus acidophilus | BCCM LMG | LMG P-26144 | 03.11.2010 | Probiotical SpA |
| 25 | Lactobacillus paracasei ssp. paracasei | BCCM LMG | LMG P-26143 | 03.11.2010 | Probiotical SpA |
| 26 | Streptococcus thermophilus | DSMZ | DSM 16506 | 18.06.2004 | Anidral Srl |

(fortgesetzt)

| Nr. | Name | Hinterlegungsstelle | Hinterlegungsnummer | Hinterlegungsdatum | Hinterleger |
|---|---|---|---|---|---|
| 27 | *Streptococcus thermophilus* | DSMZ | DSM 16507 | 18.06.2004 | Anidral Srl |
| 28 | *Streptococcus thermophilus* | DSMZ | DSM 16590 | 20.07.2004 | Anidral Srl |
| 29 | *Streptococcus thermophilus* | DSMZ | DSM 16591 | 20.07.2004 | Anidral Srl |
| 30 | *Streptococcus thermophilus* | DSMZ | DSM 16592 | 20.07.2004 | Anidral Srl |
| 31 | *Streptococcus thermophilus* | DSMZ | DSM 16593 | 20.07.2004 | Anidral Srl |
| 32 = 56 | *Bifidobacterium adolescentis* | DSMZ | DSM 16594 | 21.07.2004 | Anidral Srl |
| 33 | *Bifidobacterium adolescentis* | DSMZ | DSM 16595 | 21.07.2004 | Anidral Srl |
| 34 | *Bifidobacterium breve* | DSMZ | DSM 16596 | 21.07.2004 | Anidral Srl |
| 35 | *Bifidobacterium pseudocatenulatum* | DSMZ | DSM 16597 | 21.07.2004 | Anidral Srl |
| 36 | *Bifidobacterium pseudocatenulatum* | DSMZ | DSM 16598 | 21.07.2004 | Anidral Srl |
| 37 | *Bifidobacterium longum* | DSMZ | DSM 16603 | 20.07.2004 | Anidral Srl |
| 38 | *Bifidobacterium breve* | DSMZ | DSM 16604 | 20.07.2004 | Anidral Srl |
| 39 | *Lactobacillus casei ssp. rhamnosus* | DSMZ | DSM 16605 | 20.07.2004 | Anidral Srl |
| 40 | *Lactobacillus delbrueckii ssp. bulgaricus* | DSMZ | DSM 16606 | 20.07.2004 | Anidral Srl |
| 41 | *Lactobacillus delbrueckii ssp. bulgaricus* | DSMZ | DSM 16607 | 20.07.2004 | Anidral Srl |
| 42 | *Staphylococcus xylosus* | DSMZ | DSM 17102 | 01.02.2005 | Anidral Srl |
| 43 = 57 | *Bifidobacterium adolescentis* | DSMZ | DSM 17103 | 01.02.2005 | Anidral Srl |
| 44 | *Lactobacillus plantarum* | DSMZ | DSM 17104 | 01.02.2005 | Anidral Srl |
| 45 | *Streptococcus thermophilus* | DSMZ | DSM 17843 | 21.12.2005 | Anidral Srl |
| 46 | *Streptococcus thermophilus* | DSMZ | DSM 17844 | 21.12.2005 | Anidral Srl |
| 47 | *Streptococcus thermophilus* | DSMZ | DSM 17845 | 21.12.2005 | Anidral Srl |
| 48 | *Lactobacillus fermentum* | DSMZ | DSM 18295 | 24.05.2006 | Anidral Srl |
| 49 | *Lactobacillus fermentum* | DSMZ | DSM 18296 | 24.05.2006 | Anidral Srl |
| 50 | *Lactobacillus fermentum* | DSMZ | DSM 18297 | 24.05.2006 | Anidral Srl |
| 51 | *Lactobacillus fermentum* | DSMZ | DSM 18298 | 24.05.2006 | Anidral Srl |

(fortgesetzt)

| Nr. | Name | Hinterlegungsstelle | Hinterlegungsnummer | Hinterlegungsdatum | Hinterleger |
|---|---|---|---|---|---|
| 52 | *Lactobacillus gasseri* | DSMZ | DSM 18299 | 24.05.2006 | Anidral Srl |
| 53 | *Lactobacillus gasseri* | DSMZ | DSM 18300 | 24.05.2006 | Anidral Srl |
| 54 | *Lactobacillus gasseri* | DSMZ | DSM 18301 | 24.05.2006 | Anidral Srl |
| 55 | *Lactobacillus gasseri* | DSMZ | DSM 18302 | 24.05.2006 | Anidral Srl |
| 56 = 32 | *Bifidobacterium adolescentis EI-3* **Bifidobacterium catenulatum sp.lpseudocatenul atum EI-3l, ID 09-255** | DSMZ | DSM 18350 | 15.06.2006 | Anidral Srl |
| 57 = 43 | *Bifidobacterium adolescentis EI-15* | DSMZ | DSM 18351 | 15.06.2006 | Anidral Srl |
| 58 | *Bifidobacterium adolescentis EI-18* **Bifidobacterium animalis subsp. lactis EI-18, ID 09-256** | DSMZ | DSM 18352 | 15.06.2006 | Anidral Srl |
| 59 | *Bifidobacterium catenulatum EI-20* | DSMZ | DSM 18353 | 15.06.2006 | Anidral Srl |
| 60 | *Streptococcus thermophilus FRai* | DSMZ | DSM 18613 | 13.09.2006 | Mofin Srl |
| 61 | *Streptococcus thermophilus LB2bi* | DSMZ | DSM 18614 | 13.09.2006 | Mofin Srl |
| 62 | *Streptococcus thermophilus LRci* | DSMZ | DSM 18615 | 13.09.2006 | Mofin Srl |
| 63 | *Streptococcus thermophilus FP4* | DSMZ | DSM 18616 | 13.09.2006 | Mofin Srl |
| 64 | *Streptococcus thermophilus ZZ5F8* | DSMZ | DSM 18617 | 13.09.2006 | Mofin Srl |
| 65 | *Streptococcus thermophilus TEO4* | DSMZ | DSM 18618 | 13.09.2006 | Mofin Srl |
| 66 | *Streptococcus thermophilus S1ci* | DSMZ | DSM 18619 | 13.09.2006 | Mofin Srl |
| 67 | *Streptococcus thermophilus 641bi* | DSMZ | DSM 18620 | 13.09.2006 | Mofin Srl |
| 68 | *Streptococcus thermophilus 277A/1ai* | DSMZ | DSM 18621 | 13.09.2006 | Mofin Srl |
| 69 | *Streptococcus thermophilus 277A/2ai* | DSMZ | DSM 18622 | 13.09.2006 | Mofin Srl |
| 70 | *Streptococcus thermophilus IDC11* | DSMZ | DSM 18623 | 13.09.2006 | Mofin Srl |
| 71 | *Streptococcus thermophilus ML3di* | DSMZ | DSM 18624 | 13.09.2006 | Mofin Srl |
| 72 | *Streptococcus thermophilus TEO3* | DSMZ | DSM 18625 | 13.09.2006 | Mofin Srl |
| 73 | *Streptococcus thermophilus G62* | DSMZ | DSM 19057 | 21.02.2007 | Mofin Srl |
| 74 | *Streptococcus thermophilus G1192* | DSMZ | DSM 19058 | 21.02.2007 | Mofin Srl |
| 75 | *Streptococcus thermophilus GB18* | DSMZ | DSM 19059 | 21.02.2007 | Mofin Srl |

| Nr. | Name | Hinterlegungsstelle | Hinterlegungsnummer | Hinterlegungsdatum | Hinterleger |
|---|---|---|---|---|---|
| 76 | *Streptococcus thermophilus* CCR21 | DSMZ | DSM 19060 | 21.02.2007 | Mofin Srl |
| 77 | *Streptococcus thermophilus* G92 | DSMZ | DSM 19061 | 21.02.2007 | Mofin Srl |
| 78 | *Streptococcus thermophilus* G69 | DSMZ | DSM 19062 | 21.02.2007 | Mofin Srl |
| 79 | *Streptococcus thermophilus* | DSMZ | DSM 19063 | 21.02.2007 | Anidral Srl |
| 80 | *Streptococcus thermophilus* | DSMZ | DSM 19064 | 21.02.2007 | Anidral Srl |
| 81 | *Streptococcus thermophilus* | DSMZ | DSM 19065 | 21.02.2007 | Anidral Srl |
| 82 | *Streptococcus thermophilus* | DSMZ | DSM 19066 | 21.02.2007 | Anidral Srl |
| 83 | *Weissella* ssp. WSP 01 | DSMZ | DSM 19067 | 21.02.2007 | Anidral Srl |
| 84 | *Weissella* ssp. WSP 02 | DSMZ | DSM 19068 | 21.02.2007 | Anidral Srl |
| 85 | *Lactobacillus* ssp. WSP 03 | DSMZ | DSM 19069 | 21.02.2007 | Anidral Srl |
| 86 | *Lactobacillus plantarum* LP 09 | DSMZ | DSM 19070 | 21.02.2007 | Anidral Srl |
| 87 | *Lactobacillus plantarum* LP 10 | DSMZ | DSM 19071 | 21.02.2007 | Anidral Srl |
| 88 | *Lactococcus lactis* | DSMZ | DSM 19072 | 21.02.2007 | Anidral Srl |
| 89 | *Lactobacillus fermentum* | DSMZ | DSM 19187 | 20.03.2007 | Anidral Srl |
| 90 | *Lactobacillus fermentum* | DSMZ | DSM 19188 | 20.03.2007 | Anidral Srl |
| 91 | *Lactobacillus casei* ssp. *rhamnosus* | DSMZ | DSM 19739 | 27.09.2007 | Anidral Srl |
| 92 | *Bifidobacterium bifidum* | DSMZ | DSM 19818 | 30.10.2007 | Anidral Srl |
| 93 | *Lactobacillus delbrueckii* subsp. *bulgaricus* LD 01 | DSMZ | DSM 19948 | 28.11.2007 | Anidral Srl |
| 94 | *Lactobacillus delbrueckii* subsp. *bulgaricus* LD 02 | DSMZ | DSM 19949 | 28.11.2007 | Anidral Srl |
| 95 | *Lactobacillus delbrueckii* subsp. *bulgaricus* LD 03 | DSMZ | DSM 19950 | 28.11.2007 | Anidral Srl |
| 96 | *Lactobacillus delbrueckii* subsp. *bulgaricus* LD 04 | DSMZ | DSM 19951 | 28.11.2007 | Anidral Srl |
| 97 | *Lactobacillus delbrueckii* subsp. *bulgaricus* LD 05 | DSMZ | DSM 19952 | 28.11.2007 | Anidral Srl |
| 98 | *Bifidobacterium pseudocatenulatum* | DSMZ | DSM 21444 | 13.05.2008 | Probiotical SpA |
| 99 | *Lactobacillus acidophilus* | DSMZ | DSM 21717 | 06.08.2008 | Probiotical SpA |
| 100 | *Lactobacillus paracasei* | DSMZ | DSM 21718 | 06.08.2008 | Probiotical SpA |

(fortgesetzt)

| Nr. | Name | Hinterlegungsstelle | Hinterlegungsnummer | Hinterlegungsdatum | Hinterleger |
|---|---|---|---|---|---|
| 101 | *Lactobacillus pentosus* | DSMZ | DSM 21980 | 14.11.2008 | Probiotical SpA |
| 102 | *Lactobacillus rahmnosus* | DSMZ | DSM 21981 | 14.11.2008 | Probiotical SpA |
| 103 | *Lactobacillus delbrueckii* ssp. *delbrueckii* | DSMZ | DSM 22106 | 10.12.2008 | Probiotical SpA |
| 104 | *Lactobacillus plantarum* | DSMZ | DSM 22107 | 10.12.2008 | Probiotical SpA |
| 105 | *Lactobacillus salivarius* | DSMZ | DSM 22775 | 23.07.2009 | Probiotical SpA |
| 106 | *Lactobacillus salivarius* | DSMZ | DSM 22776 | 23.07.2009 | Probiotical SpA |
| 107 | *Bifidobacterium bifidum* | DSMZ | DSM 22892 | 28.08.2009 | Probiotical SpA |
| 108 | *Bifidobacterium bifidum* | DSMZ | DSM 22893 | 28.08.2009 | Probiotical SpA |
| 109 | *Bifidobacterium bifidum* | DSMZ | DSM 22894 | 28.08.2009 | Probiotical SpA |
| 110 | *Bifidobacterium lactis* | DSMZ | DSM 23032 | 13.10.2009 | Probiotical SpA |
| 111 | *Lactobacillus acidophilus* | DSMZ | DSM 23033 | 13.10.2009 | Probiotical SpA |
| 112 | *Lactobacillus brevis* | DSMZ | DSM 23034 | 13.10.2009 | Probiotical SpA |
| 113 | *Bifidobacterium animalis* ssp. *lactis* | DSMZ | DSM 23224 | 12.01.2010 | Probiotical SpA |
| 114 | *Bifidobacterium longum* | DSMZ | DSM 23233 | 12.01.2010 | Probiotical SpA |
| 115 | *Bifidobacterium longum* | DSMZ | DSM 23234 | 12.01.2010 | Probiotical SpA |
| 116 | *Bifidobacterium bifidum* | DSMZ | DSM 23731 | 29.06.2010 | Probiotical SpA |
| 117 | *Bifidobacterium breve* | DSMZ | DSM 23732 | 29.06.2010 | Probiotical SpA |
| 118 | *Bifidobacterium lactis* | DSMZ | DSM 23733 | 29.06.2010 | Probiotical SpA |
| 119 | *Lactobacillus reuteri* | DSMZ | DSM 23877 | 05.08.2010 | Probiotical SpA |
| 120 | *Lactobacillus reuteri* | DSMZ | DSM 23878 | 05.08.2010 | Probiotical SpA |
| 121 | *Lactobacillus reuteri* | DSMZ | DSM 23879 | 05.08.2010 | Probiotical SpA |
| 122 | *Lactobacillus reuteri* | DSMZ | DSM 23880 | 05.08.2010 | Probiotical SpA |
| 123 | *Lactobacillus paracasei* ssp. *paracasei* | DSMZ | DSM 24243 | 23.11.2010 | Probiotical SpA |
| 124 | *Lactobacillus acidophilus* | DSMZ | DSM 24303 | 23.11.2010 | Probiotical SpA |
| 125 | *Bifidobacterium bifidum* | DSMZ | DSM 24437 | 04.01.2011 | Probiotical SpA |

(fortgesetzt)

| Nr. | Name | Hinterlegungsstelle | Hinterlegungsnummer | Hinterlegungsdatum | Hinterleger |
|---|---|---|---|---|---|
| 126 | *Lactobacillus crispatus* | DSMZ | DSM 24438 | 04.01.2011 | Probiotical SpA |
| 127 | *Lactobacillus crispatus* | DSMZ | DSM 24439 | 04.01.2011 | Probiotical SpA |
| 128 | *Lactobacillus paracasei* | DSMZ | DSM 24440 | 04.01.2011 | Probiotical SpA |
| 129 | *Lactobacillus salivarius* | DSMZ | DSM 24441 | 04.01.2011 | Probiotical SpA |
| 130 | *Lactobacillus gasseri* | DSMZ | DSM 24512 | 25.01.2011 | Probiotical SpA |
| 131 | *Lactobacillus acidophilus* | DSMZ | DSM 24513 | 25.01.2011 | Probiotical SpA |
| 132 | *Lactobacillus salivarius* | DSMZ | DSM 24618 | 02.03.2011 | Probiotical SpA |
| 133 | *Lactobacillus crispatus* | DSMZ | DSM 24619 | 02.03.2011 | Probiotical SpA |
| 134 | *Lactobacillus crispatus* | DSMZ | DSM 24620 | 02.03.2011 | Probiotical SpA |
| 135 | *Lacotbacillus acidophilus* | DSMZ | DSM 24621 | 02.03.2011 | Probiotical SpA |
| 136 | *Lactobacillus gasseri* | DSMZ | DSM 24622 | 02.03.2011 | Probiotical SpA |
| 137 | *Lactobacillus paracasei* | DSMZ | DSM 24623 | 02.03.2011 | Probiotical SpA |
| 138 | *Bifidobacterium infantis* | DSMZ | DSM 24687 | 29.03.2011 | Probiotical SpA |
| 139 | *Bifidobacterium bifidum* | DSMZ | DSM 24688 | 29.03.2011 | Probiotical SpA |
| 140 | *Bifidobacterium longum* | DSMZ | DSM 24689 | 29.03.2011 | Probiotical SpA |
| 141 | *Bifidobacterium lactis* | DSMZ | DSM 24690 | 29.03.2011 | Probiotical SpA |
| 142 | *Bifidobacterium longum* | DSMZ | DSM 24691 | 29.03.2011 | Probiotical SpA |
| 143 | *Bifidobacterium breve* | DSMZ | DSM 24706 | 07.04.2011 | Probiotical SpA |
| 144 | *Bifidobacterium breve* | DSMZ | DSM 24707 | 07.04.2011 | Probiotical SpA |
| 145 | *Bifidobacterium breve* | DSMZ | DSM 24708 | 07.04.2011 | Probiotical SpA |
| 146 | *Bifidobacterium longum* | DSMZ | DSM 24709 | 07.04.2011 | Probiotical SpA |
| 147 | *Lactobacillus salivarius* | DSMZ | DSM 25138 | 02.09.2011 | Probiotical SpA |
| 148 | *Lactobacillus reuteri* | DSMZ | DSM 25139 | 02.09.2011 | Probiotical SpA |
| 149 | *Lactobacillus reuteri* | DSMZ | DSM 25140 | 02.09.2011 | Probiotical SpA |
| 150 | *Lactobacillus reuteri* | DSMZ | DSM 25141 | 02.09.2011 | Probiotical SpA |

(fortgesetzt)

| Nr. | Name | Hinterlegungsstelle | Hinterlegungsnummer | Hinterlegungsdatum | Hinterleger |
|---|---|---|---|---|---|
| 151 | *Streptococcus thermophilus* | DSMZ | DSM 25246 | 19.09.2011 | Probiotical SpA |
| 152 | *Streptococcus thermophilus* | DSMZ | DSM 25247 | 19.09.2011 | Probiotical SpA |
| 153 | *Streptococcus thermophilus* | DSMZ | DSM 25282 | 20.10.2011 | Probiotical SpA |
| 154 | *Lactobacillus salivarius* | DSMZ | DSM 25545 | 12.01.2012 | Probiotical SpA |
| 155 | *Bifidobacterium longum* | DSMZ | DSM 25669 | 16.02.2012 | Probiotical SpA |
| 156 | *Bifidobacterium longum* | DSMZ | DSM 25670 | 16.02.2012 | Probiotical SpA |
| 157 | *Bifidobacterium longum* | DSMZ | DSM 25671 | 16.02.2012 | Probiotical SpA |
| 158 | *Bifidobacterium longum* | DSMZ | DSM 25672 | 16.02.2012 | Probiotical SpA |
| 159 | *Bifidobacterium longum* | DSMZ | DSM 25673 | 16.02.2012 | Probiotical SpA |
| 160 | *Bifidobacterium longum* | DSMZ | DSM 25674 | 16.02.2012 | Probiotical SpA |
| 161 | *Bifidobacterium longum* | DSMZ | DSM 25675 | 16.02.2012 | Probiotical SpA |
| 162 | *Bifidobacterium longum* | DSMZ | DSM 25676 | 16.02.2012 | Probiotical SpA |
| 163 | *Bifidobacterium longum* | DSMZ | DSM 25677 | 16.02.2012 | Probiotical SpA |
| 164 | *Bifidobacterium longum* | DSMZ | DSM 25678 | 16.02.2012 | Probiotical SpA |
| 165 | *Bifidobacterium longum* | DSMZ | DSM 25679 | 16.02.2012 | Probiotical SpA |
| 166 | *Lactobacillus johnsonii* | DSMZ | DSM 25680 | 16.02.2012 | Probiotical SpA |
| 167 | *Lactobacillus rhamnosus* | DSMZ | DSM 25681 | 16.02.2012 | Probiotical SpA |
| 168 | *Lactobacillus rhamnosus* | DSMZ | DSM 25682 | 16.02.2012 | Probiotical SpA |
| 169 | *Lactobacillus reuteri* | DSMZ | DSM 25683 | 16.02.2012 | Probiotical SpA |
| 170 | *Lactobacillus reuteri* | DSMZ | DSM 25684 | 16.02.2012 | Probiotical SpA |
| 171 | *Lactobacillus reuteri* | DSMZ | DSM 25685 | 16.02.2012 | Probiotical SpA |
| 172 | *Bifidobacterium longum* | DSMZ | DSM 25708 | 24.02.2012 | Probiotical SpA |
| 173 | *Bifidobacterium infantis* | DSMZ | DSM 25709 | 24.02.2012 | Probiotical SpA |
| 174 | *Lactobacillus plantarum* | DSMZ | DSM 25710 | 24.02.2012 | Probiotical SpA |
| 175 | *Bifidobacterium longum* | DSMZ | DSM 25717 | 01.03.2012 | Probiotical SpA |

(fortgesetzt)

| Nr. | Name | Hinterlegungsstelle | Hinterlegungsnummer | Hinterlegungsdatum | Hinterleger |
|---|---|---|---|---|---|
| 176 | *Bifidobacterium longum* | DSMZ | DSM 25718 | 01.03.2012 | Probiotical SpA |
| 177 | *Lactobacillus salivarius* | DSMZ | DSM 26036 | 06.06.2012 | Probiotical SpA |
| 178 | *Lactobacillus salivarius* | DSMZ | DSM 26037 | 06.06.2012 | Probiotical SpA |
| 179 | *Lactobacillus pentosus* | DSMZ | DSM 26038 | 06.06.2012 | Probiotical SpA |
| 180 | *Bifidobacterium pseudolongum* ssp. *globosum* | DSMZ | DSM 26456 | 02.10.2012 | Probiotical SpA |
| 181 | *Lactobacillus fermentum* | DSMZ | DSM 26955 | 01.03.2013 | Probiotical SpA |
| 182 | *Lactobacillus fermentum* | DSMZ | DSM 26956 | 01.03.2013 | Probiotical SpA |
| 183 | *Lactobacillus casei* | DSMZ | DSM 27537 | 24.07.2013 | Probiotical SpA |
| 184 | *Lactobacillus crispatus* | DSMZ | DSM 27538 | 24.07.2013 | Probiotical SpA |
| 185 | *Lactobacillus jensenii* | DSMZ | DSM 27539 | 24.07.2013 | Probiotical SpA |

2. Verfahren nach Anspruch 1, wobei der als Toxizitätsmarker verwendete probiotische Bakterienstamm ausgewählt wird aus:

| 9 | *Lactobacillus pentosus* 9/1 ei | BCCM LMG | LMG P-21019 | 16.10.2001 | Mofin Srl |
|---|---|---|---|---|---|
| 15 | *Lactobacillus* aus der *acidophilus* Gruppe 192A/1 aiai | BCCM LMG | LMG P-21381 | 31.01.2002 | Anidral Srl |
| 17 | *Bifidobacterium breve* 195A/1 aici | BCCM LMG | LMG P-21383 | 31.01.2002 | Anidral Srl |
| 18 | *Bifidobacterium lactis* 32A/3 aiai | BCCM LMG | LMG P-21384 | 31.01.2002 | Anidral Srl |
| 22 | *Lactococcus lactis* ssp. *lactis* 501/4 ci | BCCM LMG | LMG P-21388 | 31.01.2002 | Mofin Srl |
| 33 | *Bifidobacterium adolescentis* | DSMZ | DSM 16595 | 21.07.2004 | Anidral Srl |
| 39 | *Lactobacillus casei* ssp. *rhamnosus* | DSMZ | DSM 16605 | 20.07.2004 | Anidral Srl |
| 41 | *Lactobacillus delbrueckii* ssp. *bulgaricus* | DSMZ | DSM 16607 | 20.07.2004 | Anidral Srl |
| 46 | *Streptococcus thermophilus* | DSMZ | DSM 17844 | 21.12.2005 | Anidral Srl |
| 54 | *Lactobacillus gasseri* | DSMZ | DSM 18301 | 24.05.2006 | Anidral Srl |
| 59 | *Bifidobacterium catenulatum* EI-20 | DSMZ | DSM 18353 | 15.06.2006 | Anidral Srl |
| 60 | *Streptococcus thermophilus* FRai | DSMZ | DSM 18613 | 13.09.2006 | Mofin Srl |
| 73 | *Streptococcus thermophilus* G62 | DSMZ | DSM 19057 | 21.02.2007 | Mofin Srl |
| 74 | *Streptococcus thermophilus* G1192 | DSMZ | DSM 19058 | 21.02.2007 | Mofin Srl |
| 84 | *Weissella* ssp. WSP 02 | DSMZ | DSM 19068 | 21.02.2007 | Anidral Srl |
| 95 | *Lactobacillus delbrueckii* subsp. *bulgaricus* LD 03 | DSMZ | DSM 19950 | 28.11.2007 | Anidral Srl |
| 98 | *Bifidobacterium pseudocatenulatum* | DSMZ | DSM 21444 | 13.05.2008 | Probiotical SpA |
| 101 | *Lactobacillus pentosus* | DSMZ | DSM 21980 | 14.11.2008 | Probiotical SpA |
| 105 | *Lactobacillus salivarius* | DSMZ | DSM 22775 | 23.07.2009 | Probiotical SpA |
| 116 | *Bifidobacterium bifidum* | DSMZ | DSM 23731 | 29.06.2010 | Probiotical SpA |
| 121 | *Lactobacillus reuteri* | DSMZ | DSM 23879 | 05.08.2010 | Probiotical SpA |
| 130 | *Lactobacillus gasseri* | DSMZ | DSM 24512 | 25.01.2011 | Probiotical SpA |

(fortgesetzt)

| 138 | *Bifidobacterium infantis* | DSMZ | DSM 24687 | 29.03.2011 | Probiotical SpA |
|-----|---------------------------|------|-----------|------------|-----------------|
| 143 | *Bifidobacterium breve* | DSMZ | DSM 24706 | 07.04.2011 | Probiotical SpA |
| 145 | *Bifidobacterium breve* | DSMZ | DSM 24708 | 07.04.2011 | Probiotical SpA |
| 169 | *Lactobacillus reuteri* | DSMZ | DSM 25683 | 16.02.2012 | Probiotical SpA |
| 174 | *Lactobacillus plantarum* | DSMZ | DSM 25710 | 24.02.2012 | Probiotical SpA |
| 182 | *Lactobacillus fermentum* | DSMZ | DSM 26956 | 01.03.2013 | Probiotical SpA |
| 185 | *Lactobacillus jensenii* | DSMZ | DSM 27539 | 24.07.2013 | Probiotical SpA |

3. Verfahren nach Anspruch 1 oder 2, wobei die Zählung der Bakterien mithilfe einer Methode durchgeführt wird, die einen Schritt einer Resuspendierung der ursprünglichen Probe, einen Schritt der Durchführung serieller Verdünnungen in einem geeigneten Verdünnungsmittel, einen Schritt der Ausplattierung auf einem Agar-Medium und einen Schritt des Zählens der Kolonien nach Inkubation unter optimalen Bedingungen umfasst.

4. Verfahren nach einem der vorhergehenden Ansprüche, wobei das Verhältnis zwischen der Bakterienzahl (Anzahl der gezählten Zellen auf der Platte) der ersten Testprobe und der Bakterienzahl (Anzahl der gezählten Zellen auf der Platte) der zweiten Testprobe (interne Referenz) bestimmt wird.

5. Verfahren nach einem der vorhergehenden Ansprüche, wobei die probiotischen Bakterienstämme, die als Marker für die Toxizität gegenüber probiotischen Bakterien verwendet werden, aus Lactobazillen und Bifidobakterien ausgewählt werden.

6. Verfahren nach einem der vorhergehenden Ansprüche, wobei der Grundstoff für Nahrungsmittel oder Arzneimittel aus der Gruppe ausgewählt wird, die Aromastoffe, Extrakte, Co-Formulierungsmittel organischen und/oder anorganischen Ursprungs, Vitamine, Proteine, Aminosäuren, Peptone und natürliche und/oder synthetische Polymere umfasst.

7. Verfahren zur Herstellung von Nahrungsmittelprodukten oder diätetischen Ergänzungsmitteln oder Medizinprodukten oder pharmazeutischen Produkten, die Grundstoffe, die gegenüber den ausgewählten probiotischen Bakterien, wie in Anspruch 1 definiert, nicht toxisch sind, umfassen, wobei dieses Verfahren das Verfahren zur Bestimmung der Toxizität eines Grundstoffs für ein Nahrungsmittel oder Arzneimittel nach einem oder mehreren der vorhergehenden Ansprüche 1 bis 6 umfasst.

**Revendications**

1. Procédé pour déterminer la toxicité d'une matière première alimentaire ou pharmaceutique qui comprend une première étape dans laquelle une matière première alimentaire ou pharmaceutique est placée au contact d'une charge bactérienne préétablie issue d'une souche bactérienne probiotique utilisée comme marqueur de toxicité et une seconde étape de détermination de la réduction de ladite charge bactérienne due à la toxicité exercée par ladite matière première alimentaire ou pharmaceutique vis-à-vis de ladite souche bactérienne probiotique, dans lequel ladite première étape comprend la préparation :

   - d'un premier échantillon d'essai comprenant le marqueur de souche bactérienne probiotique à une concentration préétablie, de préférence à une concentration comprise entre $1 \times 10^6$ à $1 \times 10^9$ CFU/g, le substrat de culture optimal pour la croissance de ladite souche bactérienne probiotique et la matière première alimentaire ou pharmaceutique à analyser, et

- d'un second échantillon d'essai (référence interne) comprenant la même souche bactérienne probiotique que le premier échantillon à la même concentration préétablie et le substrat de culture optimal pour la croissance de ladite souche bactérienne probiotique et dans lequel la seconde étape comprend la réalisation d'un comptage bactérien sur ledit premier et ledit second échantillon d'essai pour obtenir le nombre de bactéries ayant survécu au contact de la matière première et pour exprimer la mortalité en % induite par ladite matière première dans la souche probiotique utilisée comme marqueur, dans lequel la souche probiotique utilisée comme marqueur de toxicité est choisie parmi

Tableau 1

| No. | Nom | Institut de dépôt | Numéro de dépôt | Date de dépôt | Déposant |
|---|---|---|---|---|---|
| 1 | *Lactobacillus casei* | CNCMI.P. | I-785 | 21.07.1988 | Anidral Srl |
| 2 | *Lactobadllus gasseri* | CNCM I.P. | I-786 | 21.07.1988 | Anidral Srl |
| 3 | *Lactobacillus crispatus* | CNCM I.P. | I-787 | 21.07.1988 | Anidral Srl |
| 4 | *Lactobacillus fermentum* | CNCMI.P. | I-788 | 21.07.1988 | Anidral Srl |
| 5 | *Lactobacillus fermentum* | CNCM I.P. | I-789 | 21.07.1988 | Anidral Srl |
| 6 | *Lactobacillus casei* ssp. *pseudoplantarum* | CNCMI.P. | I-790 | 21.07.1988 | Anidral Srl |
| 7 | *Streptococcus thermophilus* B39 | BCCM LMG | LMG P-18383 | 5.05.1998 | Anidral Srl |
| 8 | *Streptococcus thermophilus* T003 | BCCM LMG | LMG P-18384 | 5.05.1998 | Anidral Srl |
| 9 | *Lactobacillus* pentosus 9 /1ei | BCCM LMG | LMG P-21019 | 16.10.2001 | Mofin Srl |
| 10 | *Lactobacillus plantarum* 776 /1 bi | BCCM LMG | LMG P-21020 | 16.10.2001 | Mofin Srl |
| 11 | *Lactobacillus plantarum* 476LL 20 bi | BCCM LMG | LMG P-21021 | 16.10.2001 | Mofin Srl |
| 12 | *Lactobacillus plantarum* PR ci | BCCM LMG | LMG P-21022 | 16.10.2001 | Mofin Srl |
| 13 | *Lactobacillus plantarum* 776 /2 hi | BCCM LMG | LMG P-21023 | 16.10.2001 | Mofin Srl |
| 14 | *Lactobacillus casei* ssp. *paracasei* 181A/3 aiai | BCCM LMG | LMG P-21380 | 31.01.2002 | Anidral Srl |
| 15 | *Lactobacillus* belonging to the *acidophilus* group 192A/1 aiai | BCCM LMG | LMG P-21381 | 31.01.2002 | Anidral Srl |
| 16 | *Bifidobacterium longum* 175A/1 aiai | BCCM LMG | LMG P-21382 | 31.01.2002 | Anidral Srl |
| 17 | *Bifidobacterium breve* 195A/1 aici | BCCM LMG | LMG P-21383 | 31.01.2002 | Anidral Srl |
| 18 | *Bifidobacterium* lactis 32A/3 aiai | BCCM LMG | LMG P-21384 | 31.01.2002 | Anidral Srl |
| 19 | *Lactobacillus plantarum* 501/2 gi | BCCM LMG | LMG P-21385 | 31.01.2002 | Mofin Srl |
| 20 | *Lactococcus lactis* ssp. *lactis* 501/4 ci | BCCM LMG | LMG P-21388 | 31.01.2002 | Mofin Srl |
| 21 | *Lactococcus lactis* ssp. *lactis* 501/4 hi | BCCM LMG | LMG P-21387 | 15.03.2002 | Mofin Srl |

(suite)

| No. | Nom | Institut de dépôt | Numéro de dépôt | Date de dépôt | Déposant |
|---|---|---|---|---|---|
| 22 | *Lactococcus lactis* ssp. *lactis* 501/4 ci | BCCM LMG | LMG P-21388 | 31.01.2002 | Mofin Srl |
| 23 | *Lactobacillus plantarum* 501/4 li | BCCM LMG | LMG P-21389 | 15.032002 | Mofin Srl |
| 24 | *Lactobacillus* acidophilus | BCCM LMG | LMG P-26144 | 03.11.2010 | Probiotical SpA |
| 25 | *Lactobacillus paracasei* ssp. *paracasei* | BCCM LMG | LMG P-26143 | 03.11.2010 | Probiotical SpA |
| 26 | *Streptococcus thermophilus* | DSMZ | DSM 16506 | 18.06.2004 | Anidral Srl |
| 27 | *Streptococcus thermophilus* | DSMZ | DSM 16507 | 18.06.2004 | Anidral Srl |
| 28 | *Streptococcus thermophilus* | DSMZ | DSM 16590 | 20.07.2004 | Anidral Srl |
| 29 | *Streptococcus thermophilus* | DSMZ | DSM 16591 | 20.07.2004 | Anidral Srl |
| 30 | *Streptococcus thermophilus* | DSMZ | DSM 16592 | 20.07.2004 | Anidral Srl |
| 31 | *Streptococcus thermophlius* | DSMZ | DSM 16593 | 20.07.2004 | Anidral Srl |
| 32 = 56 | *Bifidobacterium adolescentis* | DSMZ | DSM 16594 | 21.07.2004 | Anidral Srl |
| 33 | *Bifidobacterium adolescentis* | DSMZ | DSM 16595 | 21.07.2004 | Anidral Srl |
| 34 | *Bifidobacterium breve* | DSMZ | DSM 16596 | 21.07.2004 | Anidral Srl |
| 35 | *Bifidobacterium pseudocatenulatum* | DSMZ | DSM 16597 | 21.07.2004 | Anidral Srl |
| 36 | *Bifidobacterium pseudocatenulatum* | DSMZ | DSM 16598 | 21.07.2004 | Anidral Srl |
| 37 | *Bifidobacterium longum* | DSMZ | DSM 16603 | 20.07.2004 | Anidral Srl |
| 38 | *Bifidobacterium breve* | DSMZ | DSM 16604 | 20.07.2004 | Anidral Srl |
| 39 | *Lactobacillus casei* ssp. *rhamnosus* | DSMZ | DSM16605 | 20.07.2004 | Anidral Srl |
| 40 | *Lactobacillus delbrueckii* ssp. *bulgaricus* | DSMZ | DSM 16606 | 20.07.2004 | Anidral Srl |
| 41 | *Lactobacillus delbrueckii* ssp. *bulgaricus* | DSMZ | DSM 16607 | 20.07.2004 | Anidral Srl |
| 42 | *Staphylococcus xylosus* | DSMZ | DSM 17102 | 01.02.2005 | Anidral Srl |
| 43 = 57 | *Bifidobacterium adolescente* | DSMZ | DSM 171103 | 01.02.2005 | Anidral Srl |
| 44 | *Lactobacillus plantarum* | DSMZ | DSM 17104 | 01.02.2005 | Anidral Srl |
| 45 | *Streptococcus thermophilus* | DSMZ | DSM 17843 | 21.12.2005 | Anidral Srl |
| 46 | *Streptococcus thermophilus* | DSMZ | DSM 17844 | 21.12.2005 | Anidral Srl |
| 47 | *Streptococcus thermophilus* | DSMZ | DSM 17845 | 21.12.2005 | Anidral Sd |
| 48 | *Lactobacillus fermentum* | DSMZ | DSM 18295 | 24.05.2006 | Anidral Srl |
| 49 | *Lactobacillus fermentum* | DSMZ | DSM 18296 | 24.05.2006 | Anidral Srl |
| 50 | *Lactobacillus fermentum* | DSMZ | DSM 18297 | 24.05.2006 | Anidral Srl |
| 51 | *Ladobacillus fermentum* | DSMZ | DSM 18298 | 24.05.2006 | Anidral Srl |
| 52 | *Lactobacillus gasseri* | DSMZ | DSM 18299 | 24.05.2006 | Anidral Srl |
| 53 | *Lactobacillus gasseri* | DSMZ | DSM 18300 | 24.05.2006 | Anidral Srl |
| 54 | *Lactobacillus gasseri* | DSMZ | DSM 18301 | 24.05.2006 | Anidral Srl |

(suite)

| No. | Nom | Institut de dépôt | Numéro de dépôt | Date de dépôt | Déposant |
|---|---|---|---|---|---|
| 55 | *Lactobacillus gasseri* | DSMZ | DSM 18302 | 24.05.2006 | Anidral Srl |
| 56 = 32 | *Bifidobacterium adolescentis* EI-3 **Bifidobacterium catenulatum sp./ pseudocatenul atum EI-3I, ID 09-255** | DSMZ | DSM 18350 | 15.06.2006 | Anidral Srl |
| 57 =43 | *Bifidobacterium adolescentis* EI-15 | DSMZ | DSM 18351 | 15.06.2006 | Anidral Srl |
| 58 | *Bifidobacterium adolescentis* EI-18 **Bifidobacterium animalis subsp. lactis EI-18, ID 09-256** | DSMZ | DSM 18352 | 15.06.2006 | Anidral Srl |
| 59 | *Bifidobacterium catenulatum* EI-20 | DSMZ | DSM 18353 | 15.06.2006 | Anidral Srl |
| 60 | *Streptococcus thermophilus* FRai | DSMZ | DSM 18613 | 13.09.2006 | Mofin Srl |
| 61 | *Streptococcus thermophilus* LB2bi | DSMZ | DSM 18614 | 13.09.2006 | Mofin Srl |
| 62 | *Streptococcus thermophilus* LRci | DSMZ | DSM 18615 | 13.09.2006 | Mofin Srl |
| 63 | *Streptococcus thermophilus* FP4 | DSMZ | DSM 18616 | 13.09.2006 | Mofin Srl |
| 64 | *Streptococcus thermophilus* ZZ5F8 | DSMZ | DSM 18617 | 13.09.2006 | Mofin Srl |
| 65 | *Streptococcus thermophilus* TEO4 | DSMZ | DSM 18618 | 13.09.2006 | Mofin Srl |
| 66 | *Streptococcus thermiphilus* S1ci | DSMZ | DSM 18619 | 13.09.2006 | Mofin Srl |
| 67 | *Streptococcus thermophilus* 641 bi | DSMZ | DSM 18620 | 13.09.2006 | Mofin Srl |
| 68 | Streptococcus *thermophilus* 277A/1ai | DSMZ | DSM 18621 | 13.09.2006 | Mofin Srl |
| 69 | *Streptococcus thermophilus* 277A/2ai | DSMZ | DSM 18622 | 13.09.2006 | Mofin Srl |
| 70 | *Streptococcus thermophilus* IDC11 | DSMZ | DSM 18623 | 13.09.2006 | Mofin Srl |
| 71 | *Streptococcus thermophilus* ML3di | DSMZ | DSM 18624 | 13.09.2006 | Mofin Srl |
| 72 | *Streptococcus thermophilus* TEO3 | DSMZ | DSM 18625 | 13.09.2006 | Mofin Srl |
| 73 | *Streptococcus thermophilus* G62 | DSMZ | DSM 19057 | 21.02.2007 | Mofin Srl |
| 74 | *Streptococcus thermophilus* G1192 | DSMZ | DSM 19058 | 21.02.2007 | Mofin Srl |
| 75 | *Streptococcus thermophilus* GB18 | DSMZ | DSM 19059 | 21.02.2007 | Mofin Srl |
| 76 | *Streptococcus thermophilus* CCR21 | DSMZ | DSM 19060 | 21.02.2007 | Mofin Srl |
| 77 | *Streptococcus thermophilus* G92 | DSMZ | DSM 19061 | 21.02.2007 | Mofin Srl |
| 78 | *Streptococcus thermophilus* G69 | DSMZ | DSM 19062 | 21.02.2007 | Mofin Srl |
| 79 | *Streptococcus thermophilus* | DSMZ | DSM 19063 | 21.02.2007 | Anidral Srl |
| 80 | *Streptococcus thermophilus* | DSMZ | DSM 19064 | 21.02.2007 | Anidral Srl |
| 81 | *Streptococcus thermophilus* | DSMZ | DSM 19065 | 21.02.2007 | Anidral Srl |
| 82 | *Streptococcus thermophilus* | DSMZ | DSM 19066 | 21.02.2007 | Anidral Srl |
| 83 | *Weissella* ssp. WSP 01 | DSMZ | DSM 19067 | 21.02.2007 | Anidral Srl |
| 84 | *Weissella* ssp. WSP 02 | DSMZ | DSM 19068 | 21.02.2007 | Anidral Srl |
| 85 | *Lactobacillus* ssp. WSP 03 | DSMZ | DSM 19069 | 21.02.2007 | Anidral Srl |
| 86 | *Lactobacillus plantarum* LP 09 | DSMZ | DSM 19070 | 21.02.2007 | Anidral Srl |
| 87 | *Lactobacillus plantarum* LP 10 | DSMZ | DSM 19071 | 21.02.2007 | Anidral Srl |

(suite)

| No. | Nom | Institut de dépôt | Numéro de dépôt | Date de dépôt | Déposant |
|---|---|---|---|---|---|
| 88 | *Lactococcus lactis* | DSMZ | DSM 19072 | 21.02.2007 | Anidral Srl |
| 89 | *Lactobacillus fermentum* | DSMZ | DSM 19187 | 20.03.2007 | Anidral Srl |
| 90 | *Lactobacillus fermentum* | DSMZ | DSM 19188 | 20.03.2007 | Anidral Srl |
| 91 | *Lactobacillus casei* ssp. *rhamnosus* | DSMZ | DSM 19739 | 27.09.2007 | Anidral Srl |
| 92 | *Bifidobacterium bifidum* | DSMZ | DSM 19818 | 30.10.2007 | Anidral Srl |
| 93 | *Lactobacillus delblrueckii* subsp. *bulgaricus* LD 01 | DSMZ | DSM 19948 | 28.11.2007 | Anidral Srl |
| 94 | *Lactobacillus delbrueckii* subsp. *bulgaricus* LD 02 | DSMZ | DSM 19949 | 28.11.2007 | Anidral Srl |
| 95 | *Lactobacillus delbrueckii* subsp. *bulgaricus* LD 03 | DSMZ | DSM 19950 | 28.11.2007 | Anidral Srl |
| 96 | *Lactobacillus delbrueckii* subsp. *bulgaricus* LD 04 | DSMZ | DSM 19951 | 28.11.2007 | Anidral Srl |
| 97 | *Lactobacillus delbrueckii* subsp. *bulgaricus* LD 05 | DSMZ | DSM 19952 | 28.11.2007 | Anidral Srl |
| 98 | *Bifidobacterium pseudocatenulatum* | DSMZ | DSM 21444 | 13.05.2008 | Probiotical SpA |
| 99 | *Lactobacillus acidophilus* | DSMZ | DSM 21717 | 06.08.2008 | Probiotical SpA |
| 100 | *Lactobacillus paracasei* | DSMZ | DSM 21718 | 06.08.2008 | Probiotical SpA |
| 101 | *Lactobacillus pentosus* | DSMZ | DSM 21980 | 14.11.2008 | Probiotical SpA |
| 102 | *Lactobacillus rahmnosus* | DSMZ | DSM 21981 | 14.11.2008 | Probiotical SpA |
| 103 | *Lactobacillus delbrueckii* ssp. *delbrueckii* | DSMZ | DSM 22106 | 10.12.2008 | Probiotical SpA |
| 104 | *Lactobacillus plantarum* | DSMZ | DSM 22107 | 10.12.2008 | Probiotical SpA |
| 105 | *Lactobacillus salivarius* | DSMZ | DSM 22775 | 23.07.2009 | Probiotical SpA |
| 106 | *Lactobacillus salivarius* | DSMZ | DSM 22776 | 23.07.2009 | Probiotical SpA |
| 107 | *Bifidobacterium bifidum* | DSMZ | DSM 22892 | 28.08.2009 | Probiotical SpA |
| 108 | *Bifidobacterium bifidum* | DSMZ | DSM 22893 | 28.08.2009 | Probiotical SpA |
| 109 | *Bifidobacterium bifidum* | DSMZ | DSM 22894 | 28.08.2009 | Probiotical SpA |
| 110 | *Bifidobacterium lactis* | DSMZ | DSM 23032 | 13.10.2009 | Probiotical SpA |
| 111 | *Lactobacillus acidophilus* | DSMZ | DSM 23033 | 13.10.2009 | Probiotical SpA |
| 112 | *Lactobacillus brevis* | DSMZ | DSM 23034 | 13.10.2009 | Probiotical SpA |
| 113 | *Bifidobacterium animalis* ssp. *lectis* | DSMZ | DSM 23224 | 12.01.2010 | Probiotical SpA |

(suite)

| No. | Nom | Institut de dépôt | Numéro de dépôt | Date de dépôt | Déposant |
|---|---|---|---|---|---|
| 114 | *Bifidobacterium longum* | DSMZ | DSM 23233 | 12.01.2010 | Probiotical SpA |
| 115 | *Bifidobacterium longum* | DSMZ | DSM 23234 | 12.01.2010 | Probiotical SpA |
| 116 | *Bifidobacterium bifidum* | DSMZ | DSM 23731 | 29.06.2010 | Probiotical SpA |
| 117 | *Bifidobacterium breve* | DSMZ | DSM 23732 | 29.06.2010 | Probiotical SpA |
| 118 | *Bifidobacterium lactis* | DSMZ | DSM 23733 | 29.06.2010 | Probiotical SpA |
| 119 | *Lactobacillus reuteri* | DSMZ | DSM 23877 | 05.08.2010 | Probiotical SpA |
| 120 | *Lactobacillus reuteri* | DSMZ | DSM 23878 | 05.08.2010 | Probiotical SpA |
| 121 | *Lactobacillus reuteri* | DSMZ | DSM 23879 | 05.08.2010 | Probiotical SpA |
| 122 | *Lactobacilius reuteri* | DSMZ | DSM 23880 | 05.08.2010 | Probiotical SpA |
| 123 | *Lactobacillus paracasei* ssp. *paracasei* | DSMZ | DSM 24243 | 23.11.2010 | Probiotical SpA |
| 124 | *Lactobacillus acidophilus* | DSMZ | DSM 24303 | 2311.2010 | Probiotical SpA |
| 125 | *Bifldobacterium bifidum* | DSMZ | DSM 24437 | 04.01.2011 | Probiotical SpA |
| 126 | *Ladobacillus crispatus* | DSMZ | DSM 24438 | 04.01.2011 | Probiotical SpA |
| 127 | *Lactobacillus crispatus* | DSMZ | DSM 24439 | 04.01.2011 | Probiotical SpA |
| 128 | *Lactobacillus paracasei* | DSMZ | DSM 24440 | 04.01.2011 | Probiotical SpA |
| 129 | *Lactobacillus salivarius* | DSMZ | DSM 24441 | 04.01.2011 | Probiotical SpA |
| 130 | *Lactobacillus gasseri* | DSMZ | DSM 24512 | 25.01.2011 | Probiotical SpA |
| 131 | *Lactobacillus acidophilus* | DSMZ | DSM 24513 | 25.01.2011 | Probiotical SpA |
| 132 | *Lactobacillus salivarius* | DSMZ | DSM 24618 | 02.03.2011 | Probiotical SpA |
| 133 | *Laciobacillus crispatus* | DSMZ | DSM 24619 | 02.03.2011 | Probiotical SpA |
| 134 | *Lactobacillus crispatus* | DSMZ | DSM 24620 | 02.03.2011 | Probiotical SpA |
| 135 | *Lacotbacillus acidophilus* | DSMZ | DSM 24621 | 02.03.2011 | Probiotical SpA |

(suite)

| No. | Nom | Institut de dépôt | Numéro de dépôt | Date de dépôt | Déposant |
|---|---|---|---|---|---|
| 136 | *Lactobacillus gasseri* | DSMZ | DSM 24622 | 02.03.2011 | Probiotical SpA |
| 137 | *Lactobacillus paracasei* | DSMZ | DSM 24623 | 02.03.2011 | Probiotical SpA |
| 138 | *Bifidobacterium infantis* | DSMZ | DSM 24687 | 29.03.2011 | Probiotical SpA |
| 139 | *Bifidobactarium bifidum* | DSMZ | DSM 24688 | 29.03.2011 | Probiotical SpA |
| 140 | *Bifidobacterium longum* | DSMZ | DSM 24689 | 29.03.2011 | Probiotical SpA |
| 141 | *Bifidobacterium lactis* | DSMZ | DSM 24690 | 29.03.2011 | Probiotical SpA |
| 142 | *Bifidobacterium longum* | DSMZ | DSM 24691 | 29.03.2011 | Probiotical SpA |
| 143 | *Bifidobacterium breve* | DSMZ | DSM 24706 | 07.04.2011 | Probiotical SpA |
| 144 | *Bifidobacterium breve* | DSMZ | DSM 24707 | 07.04.2011 | Probiotical SpA |
| 145 | *Bifidobacterium breve* | DSMZ | DSM 24708 | 07.04.2011 | Probiotical SpA |
| 146 | *Bifidobacterium longum* | DSMZ | DSM 24709 | 07.04.2011 | Probiotical SpA |
| 147 | *Lactobecillus salivarius* | DSMZ | DSM 25138 | 02.09.2011 | Probiotical SpA |
| 148 | *Lactobacillus reuteri* | DSMZ | DSM 25139 | 02.09.2011 | Probiotical SpA |
| 149 | *Lactobacillus reuteri* | DSMZ | DSM 25140 | 02.09.2011 | Probiotical SpA |
| 150 | *Lactobacillus reuteri* | DSMZ | DSM 25141 | 02.092011 | Probiotical SpA |
| 151 | *Streptococcus thermophilus* | DSMZ | DSM 25246 | 19.09.2011 | Probiotical SpA |
| 152 | *Streptococcus thermophilus* | DSMZ | DSM 25247 | 19.09.2011 | Probiotical SpA |
| 153 | *Streptococcus thermophilus* | DSMZ | DSM 25282 | 20.10.2011 | Probiotical SpA |
| 154 | *Lactobacillus salivarius* | DSMZ | DSM 25545 | 12.01.2012 | Probiotical SpA |
| 155 | *Bifidobacterium longum* | DSMZ | DSM 25669 | 16.02.2012 | Probiotical SpA |
| 156 | *Bifidobacterium longum* | DSMZ | DSM 25670 | 16,02.2012 | Probiotical SpA |
| 157 | *Bifidobacterium longum* | DSMZ | DSM 25671 | 16.02.2012 | Probiotical SpA |

(suite)

| No. | Nom | Institut de dépôt | Numéro de dépôt | Date de dépôt | Déposant |
|---|---|---|---|---|---|
| 158 | *Bifidobacterium longum* | DSMZ | DSM 25672 | 16.02.2012 | Probiotical SpA |
| 159 | *Bifidobacterium longum* | DSMZ | DSM 25673 | 16.02.2012 | Probiotical SpA |
| 160 | *Bifidobacterium longum* | DSMZ | DSM 25674 | 16,02.2012 | Probiotical SpA |
| 161 | *Bifidobacterium longum* | DSMZ | DSM 25675 | 16.02.2012 | Probiotical SpA |
| 162 | *Bifidobacterium longum* | DSMZ | DSM 25676 | 16,02.2012 | Probiotical SpA |
| 163 | *Bifidobacterium longum* | DSMZ | DSM 25677 | 16.02.2012 | Probiotical SpA |
| 164 | *Bifidobacterium longum* | DSMZ | DSM 25678 | 16.02.2012 | Probiotical SpA |
| 165 | *Bifidobacterium longum* | DSMZ | DSM 25679 | 16.02.2012 | Probiotical SpA |
| 166 | *Lactobacillus johnsonii* | DSMZ | DSM 25680 | 16.02.2012 | Probiotical SpA |
| 167 | *Lactobocillus rhamnosus* | DSMZ | DSM 25681 | 16.02.2012 | Probiotical SpA |
| 168 | *Lactobacillus rhamnosus* | DSMZ | DSM 25682 | 16.02.2012 | Probiotical SpA |
| 169 | *Lactobacillus reuteri* | DSMZ | DSM 25683 | 16.02.2012 | Probiotical SpA |
| 170 | *Lactobacillus reuteri* | DSMZ | DSM 25684 | 16.02.2012 | Probiotical SpA |
| 171 | *Lactobacillus reuteri* | DSMZ | DSM 25685 | 16.02.2012 | Probiotical SpA |
| 172 | *Bifidobacterium longum* | DSMZ | DSM 25708 | 24.02.2012 | Probiotical SpA |
| 173 | *Bifidobacterium infantis* | DSMZ | DSM 25709 | 24.02.2012 | Probiotical SpA |
| 174 | *Lactobacillus plantarum* | DSMZ | DSM 25710 | 24.02.2012 | Probiotical SpA |
| 175 | *Bifidobacterium longum* | DSMZ | DSM 25717 | 01.03 2012 | Probiotical SpA |
| 176 | *Bifidobacterium longum* | DSMZ | DSM 25718 | 01.03.2012 | Probiotical SpA |
| 177 | *Lactobacillus salivarius* | DSMZ | DSM 26036 | 06.06.2012 | Probiotical SpA |
| 178 | *Lactobacillus salivarius* | DSMZ | DSM 26037 | 06.06.2012 | Probiotical SpA |
| 179 | *Lactobacillius pentosus* | DSMZ | DSM 26038 | 06.06.2012 | Probiotical SpA |

(suite)

| No. | Nom | Institut de dépôt | Numéro de dépôt | Date de dépôt | Déposant |
|---|---|---|---|---|---|
| 180 | *Bifidobacterim pseudolongum* ssp. *globosum* | DSMZ | DSM 26456 | 02.10.2012 | Probiotical SpA |
| 181 | *Lactobacillus fermentum* | DSMZ | DSM 26955 | 01.03.2013 | Probiotical SpA |
| 182 | *Lactobacillus fermentum* | DSMZ | DSM 26956 | 01.03.2013 | Probiotical SpA |
| 183 | *Lactobacillus casei* | DSMZ | DSM 27537 | 24.07.2013 | Probiotical SpA |
| 184 | *Lactobacillus crispatus* | DSMZ | DSM 27538 | 24.07.2013 | Probiotical SpA |
| 185 | *Lactobacillus jensenii* | DSMZ | DSM 27539 | 24.07.2013 | Probiotical SpA |

2. Procédé selon la revendication 1, dans lequel la souche bactérienne probiotique utilisée comme marqueur de toxicité est choisie parmi

| 9 | *Lactobacillus pentosus* 9/1 ei | BCCM LMG | LMG P-21019 | 16.10.2001 | Mofin Srl |
|---|---|---|---|---|---|
| 15 | *Lactobacillus* belonging to the *acidophilus* group 192A/1 aiai | BCCM LMG | LMG P-21381 | 31.01.2002 | Anidral Srl |
| 17 | *Bifidobacterium breve* 195A/1 aici | BCCM LMG | LMG P-21383 | 31.01.2002 | Anidral Srl |
| 18 | *Bifidobacterium lactis* 32A/3 aiai | BCCM LMG | LMG P-21384 | 31.01.2002 | Anidral Srl |
| 22 | *Lactococcus lactis* ssp. *lactis* 501/4 ci | BCCM LMG | LMG P-213.88 | 31.01.2002 | Mofin Srl |
| 33 | *Bifidobacterium adolescentis* | DSMZ | DSM 16595 | 21.07.2004 | Anidral Srl |
| 39 | *Lactobacillus casei* ssp. *rhamnosus* | DSMZ | DSM 16605 | 20.07.2004 | Anidral Srl |
| 41 | *Lactobacillus delbrueckii* ssp. *bulgaricus* | DSMZ | DSM 16607 | 20.07.2004 | Anidral Srl |
| 46 | *Streptococcus thermophilus* | DSMZ | DSM 17844 | 21.12.2005 | Anidral Srl |
| 54 | *Lactobacillus gasseri* | DSMZ | DSM 18301 | 24.05.2006 | Anidral Srl |
| 59 | *Bifidobacterium catenulatum* EI-20 | DSMZ | DSM 18353 | 15.06.2006 | Anidral Srl |
| 60 | *Streptococcus thermophilus* FRai | DSMZ | DSM 18613 | 13.09.2006 | Mofin Srl |
| 73 | *Streptococcus thermophilus* G62 | DSMZ | DSM 19057 | 21.02.2007 | Mofin Srl |

(suite)

| 74 | *Streptococcus thermophilus* G1192 | DSMZ | DSM 19058 | 21.02.2007 | Mofin Srl |
|-----|-------------------------------------|------|------------|------------|-----------|
| 84 | ***Weissella*** ssp. WSP 02 | DSMZ | DSM 19068 | 21.02.2007 | Anidral Srl |
| 95 | *Lactobacillus delbrueckii* subsp. *bulgaricus* LD 03 | DSMZ | DSM 19950 | 28.11.2007 | Anidral Srl |
| 98 | *Bifidobaderium pseudoeatenulatum* | DSMZ | DSM 21444 | 13.05.2008 | Probiotical SpA |
| 101 | *Lactobacillus pentosus* | DSMZ | DSM 21980 | 14.11.2008 | Probiotical SpA |
| 105 | *Lactobacillus salivarius* | DSMZ | DSM 22775 | 23.07.2009 | Probiotical SpA |
| 116 | *Bifidobacterium bifidum* | DSMZ | DSM 23731 | 29.06.2010 | Probiotical SpA |
| 121 | *Lactobacillus reuteri* | DSMZ | DSM 23879 | 05.08.2010 | Probiotical SpA |
| 130 | *Lactobacillus gassari* | DSMZ | DSM 24512 | 25.01.2011 | Probiotical SpA |
| 138 | *Bifidobaderium infantis* | DSMZ | DSM 24687 | 29.03.2011 | Probiotical SpA |
| 143 | *Bifidobacterium breve* | DSMZ | DSM 24706 | 07.04.2011 | Probiotical SpA |
| 145 | *Bifidobacterium breve* | DSMZ | DSM 24708 | 07.04.2011 | Probiotical SpA |
| 169 | *Lactobacillus reuteri* | DSMZ | DSM 25683 | 16.02.2012 | Probiotical SpA |
| 174 | *Lactobacillus plantarum* | DSMZ | DSM 25710 | 24.02.2012 | Probiotical SpA |
| 182 | *Lactobacillus fermentum* | DSMZ | DSM 26956 | 01.03.2013 | Probiotical SpA |
| 185 | *Lactobacillus jensenii* | DSMZ | DSM 27539 | 24.07.2013 | Probiotical SpA |

3. Procédé selon la revendication 1 ou 2, dans lequel ledit comptage bactérien est réalisé à l'aide d'un procédé comprenant une étape de remise en suspension de l'échantillon inital, une étape de préparation de dilutions en série dans un diluant convenable, une étape d'étalement en milieu agarisé et une étape de comptage des colonies après incubation dans des conditions optimales.

4. Procédé selon l'une quelconque des revendications précédentes, dans lequel le rapport entre le comptage bactérien (nombre de cellules sur la plaque) dudit premier échantillon d'essai et le comptage bactérien (nombre de cellules sur la plaque) dudit second échantillon d'essai (référence interne) est déterminé.

5. Procédé selon l'une quelconque des revendications précédentes, dans lequel les souches bactériennes probiotiques utilisées comme marqueurs de toxicité vis-à-vis des bactéries probiotiques sont choisies parmi les lactobacilles et les bifidobactéries.

6. Procédé selon l'une quelconque des revendications précédentes, dans lequel ladite matière première alimentaire ou pharmaceutique est choisie dans le groupe comprenant les aromatisants, les extraits, les co-formulants d'origine organique et/ou inorganique, les vitamines, les protéines, les acides aminés, les peptones, les polymères naturels et/ou synthétiques.

7. Procédé de production de produits alimentaires ou de compléments alimentaires ou de dispositifs médicaux ou de produits pharmaceutiques comprenant des matières premières qui ne sont pas toxiques vis-à-vis des bactéries probiotiques choisies comme défini dans la revendication 1, ledit procédé comprenant le procédé pour déterminer la toxicité d'une matière première alimentaire ou pharmaceutique selon une ou plusieurs des revendications 1 à 6 précédentes.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102304482 A

- CN 102268471 A

**Non-patent literature cited in the description**

- **SAMBROOK ; FRITSCH ; MANIATIS.** Molecular Cloning a Laboratory Manual
- **M. DANIELSEN ; A. WIND.** *Susceptibility of Lactobacillus spp. to antimicrobial agents.,* 2002
- **S. DELGRADO ; A.B. FLOREZ ; B. MAYO.** *Antibiotic Susceptibility of Lactobacillus and Bifidobacterium species from Human Gastrointestinal tract,* 2005

- **FONT DE VALDEZ, G.** *Influence of the recovery medium on the viability of injured freeze-dried lactic acid bacteria Milchwissenschaft,* 1985, vol. 40 (9), 518-520
- **LC. MCDONALD ; R.F. MCFEETERS ; M.A. DAESCHEL ; HP FELMING.** A differential medium for the enumeration of homofermentative and heterofermentative lactic acid bacteria. *Applied and Environmental Microbiology,* June 1987, 1382-1384